(19) Europäisches Patentamt
European Patent Office
Office européen des brevets

(11) EP 1 191 024 A1

(12) **EUROPEAN PATENT APPLICATION**

(43) Date of publication:
**27.03.2002 Bulletin 2002/13**

(51) Int Cl.[7]: **C07D 285/16**, C07D 417/12, A61K 31/54, A61P 37/00

(21) Application number: **00120727.3**

(22) Date of filing: **22.09.2000**

(84) Designated Contracting States:
**AT BE CH CY DE DK ES FI FR GB GR IE IT LI LU MC NL PT SE**
Designated Extension States:
**AL LT LV MK RO SI**

(71) Applicants:
- **Tschesche, Harald**
**D-33615 Bielefeld (DE)**
- **Schröder, Jörg**
**32791 Bielefeld (DE)**

(72) Inventors:
- **Tschesche, Harald**
**D-33615 Bielefeld (DE)**
- **Schröder, Jörg**
**32791 Bielefeld (DE)**

(74) Representative: **Boeters, Hans Dietrich, Dr. et al**
**Patentanwälte Boeters & Bauer,**
**Bereiteranger 15**
**81541 München (DE)**

(54) **Thiadiazines and their use as inhibitors of metalloproteinases**

(57) The present invention provides novel thiadiazines represented by formula (I) which are useful as inhibitors of metalloproteinases. Also disclosed are pharmaceutical compositions and methods of treating disorders associated with increased activity of metalloproteinases using these compounds or the pharmaceutical compositions containing them.

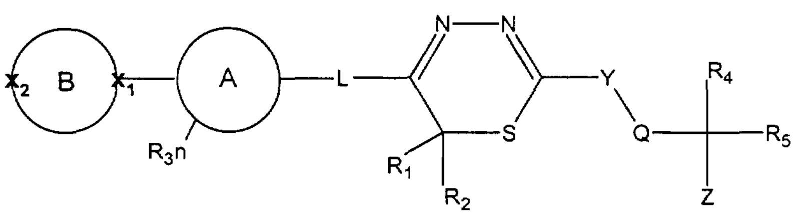

(I)

EP 1 191 024 A1

## Description

TECHNICAL FIELD

The present invention relates to compounds or pharmaceutically acceptable salts thereof, to processes for their preparation, to pharmaceutical compositions containing them, and to the use of such compounds in medicine. In particular, the compounds are inhibitors of metalloproteinases involved in tissue degradation.

BACKGROUND OF THE INVENTION

Metalloproteinases are a superfamily of enzymes whose numbers in recent years have increased dramatically. Based on structural and functional considerations they have been classified into families and subfamilies[1,2]. Examples of metalloproteinases include the matrix metalloproteinases (MMPs) which are zinc-dependent endopeptidases involved in the degradation and remodeling of connective tissues. Members of these MMPs are present in various cell types that reside in or are associated with connective tissue, such as fibroblasts, monocytes, macrophages, endothelial cells, and invasive or metastatic tumor cells. MMP expression is stimulated by growth factors and cytokines in the local tissue environment, where these enzymes act to specifically degrade protein components of the extracellular matrix, such as collagen, proteoglycans, fibronectin and laminin. These ubiquitous extracellular matrix components are present in the linings of joints, interstitial connective tissues, basement membranes and cartilage[3].

Excessive degradation of extracellular matrix by MMPs is implicated in the pathogenesis of many diseases, including rheumatoid arthritis, osteoarthritis, osteopenias such as osteoporosis, myelin degradation such as multiple sclerosis, angiogenesis dependent diseases, chronic obstructive pulmonary disease, cerebral hemorrhaging associated with stroke, periodontitis, gingivitis, corneal epidermal or gastric ulceration, tumor metastasis invasion and growth, and in complications of diabetes. Therefore the inhibition of the involved MMPs is recognized as a good target for therapeutic intervention[4,5].

The MMPs share a number of properties, including zinc and calcium dependence, secretion as zymogens, and 40-50% amino acid sequence homology. At present time, there are eighteen known human MMPs which are characterized by structural and functional properties and include gelatinases, stromelysins, collagenases, matrilysin, metalloelastase and membrane-type MMPs, as discussed in greater detail below.

The gelatinases include two distinct, but highly related enzymes: a 72-kD enzyme (HFG, MMP-2, or gelatinase A) secreted by fibroblasts and a wide variety of other cell types, and a 92-kD enzyme (HNG, MMP-9, or gelatinase B) released by mononuclear phagocytes, neutrophils, corneal epithelial cells, tumor cells, cytotrophoblasts and keratinocytes. These gelatinases have been shown to degrade gelatins (denatured collagens), collagen types IV (basement membrane) and V, fibronectin and insoluble elastin.

Stromelysins 1 and 2 have been shown to cleave a broad range of matrix substrates, including laminin, fibronectin, proteoglycans and collagen types IV and IX in their non-helical domains.

Interstitial collagenases catalyze the initial and rate-limiting cleavage of native collagen types I, II and III. Collagen, the major structural protein of mammals, is an essential matrix component of many tissues like cartilage, bone, tendon and skin, for example. Interstitial collagenases are very specific matrix metalloproteinases which cleave these collagens resulting in two fragments which spontaneously denature at physiological temperatures and therefore become susceptible to cleavage by less specific enzymes. Cleavage by the collagenases results in the loss of structural integrity of the target tissue, which is essentially an irreversible process. There are currently three known human collagenases. The first is human fibroblast collagenase (HFC, MMP-1, or collagenase-1) and is produced by a wide variety of cells including fibroblasts and macrophages. The second is human neutrophil collagenase (HNC, MMP-8, or collagenase-2) that has so far only been demonstrated to be produced by neutrophils and keratinocytes. The most recently discovered member of this group of MMPs is human collagenase-3 (MMP-13) which was originally found in breast carcinomas, but has been shown to be also produced by chondrocytes.

Matrilysin (MMP-7, PUMP-1) has been shown to degrade a wide range of matrix substrates including proteoglycans, gelatin, fibronectin, elastin, and laminin. Its expression has been documented in mononuclear phagocytes, rat uterine explants and in tumors.

Other less characterized MMPs include human macrophage elastase (MMP-12, HME), membrane type-1 MMP (MMP-14, MT-1 MMP) and stromelysin-3 (MMP-11). A recent review of matrix metalloproteinases provides an excellent source for detailed information and references on these enzymes[6].

Further examples of metalloproteinases include the reprolysin or adamalysin (ADAM) family which includes the secretases and sheddases such as TNF-$\alpha$ converting enzyme (ADAM-17); the astacin family which include enzymes such as procollagen processing proteinase (PCP); and other metalloproteinases such as aggrecanase, the endothelin converting enzyme family and the angiotensin converting enzyme family. ADAM-17, also known as tumor necrosis factor-alpha converting enzyme (TACE), is the most well known ADAM. ADAM-17 is resposible for cleavage

of cell bound tumor necrosis factor-alpha (TNF-α). TNF-α is recognized to be involved in many infectious and autoimmune diseases. Furthermore, TNF-α is the prime mediator of the inflammatory response seen in sepsis and septic shock[7]. There are two forms of TNF-α, a type II membrane protein of relative molecular mass 26 kD and a soluble 17 kD form generated from the cell bound protein by specific proteolytic cleavage. The soluble 17 kD form of TNF-α is released by the cell and is associated with the deleterious effects of TNF-α. This form of TNF-α is also capable of acting at sites distant from the site of synthesis. Thus, inhibitors of TACE prevent the formation of soluble TNF-α and prevent the deleterious effects of the soluble factor. Other ADAMs that have shown expression in pathological situations include ADAM TS-1, and ADAM-10, -12 and -15, respectively[8,9]. As knowledge of the expression, physiological substrates and disease application of the ADAMs increases, the full significance of the role of inhibition of this class of enzymes will be appreciated.

Inhibitors of metalloproteinases may provide useful treatments for diseases associated with the excessive degradation of extracellular matrix, such as arthritic diseases (rheumatoid arthritis and osteoarthritis), bone resorptive diseases (such as osteoporosis), the enhanced collagen destruction associated with diabetes, chronic obstructive pulmonary disease, cerebral hemorrhaging associated with stroke, periodontal disease, corneal or gastric ulceration, ulceration of the skin, tumor invasion and metastasis. MMP inhibitors are also of potential value in the treatment of neuroinflammatory disorders, including those involving myelin degradation, for example multiple sclerosis, as well as in the management of angiogenesis dependent diseases, which include arthritic conditions and solid tumor growth as well as psoriasis, proliferative retinopathies, neovascular glaucoma, ocular tumors, angiofibromas and hemoangiomas. However, it is recognized that different combinations of MMPs and ADAMs are expressed in different pathological situations. According to this, inhibitors with specific selectivities for individual ADAMs and/or MMPs may be preferred for individual diseases. For example, rheumatoid arthritis is an inflammatory joint disease characterized by excessive TNF-α levels and the loss of joint matrix constituents. In this case, a compound that inhibits TACE as well as MMPs such as MMP-13 may be preferred for therapeutical use. In contrast, in less inflammatory joint disease such as osteoarthritis, compounds that inhibit matrix degrading MMPs such as MMP-13 but not TACE may be preferred.

Many known MMP inhibitors are peptide derivatives, based on naturally occurring amino acids, and are analogues of the cleavage site in the collagen molecule[10,11]. Other known MMP inhibitors are less peptidic in structure, and may more properly be viewed as pseudopeptides or peptide mimetics. Such compounds usually have a functional group capable of binding to the zinc(II)site in the MMP which is known to be the catalytic center, and known classes include those in which the zinc binding group is a hydroxamic acid, carboxylic acid, sulphydryl, 5-thioxo-1,3,4-thiadiazole, and oxygenated phosphorus group. Known compounds show potent *in vitro* activities, but are generally poorly absorbed following oral administration. It is known that a number of factors can influence oral absorption (such as aqueous solubility, $pK_a$, log p and molecular weight), and the design of pseudo- or non-peptide enzyme inhibitors with high oral absorption is far from straightforward[12,13].

The design and use of metalloproteinase inhibitors are the subject of numerous patents and patent applications[14]. The value of thiadiazines as inhibitors of metalloproteinases has not hitherto been recognized however. Moreover, the compounds claimed in this invention are apparently novel and have not been previously described in the literature.

## SUMMARY OF THE INVENTION

In a first aspect of the invention we provide compounds represented by formula (I):

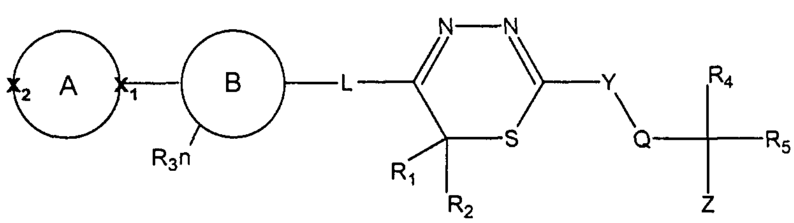

**(I)**

wherein:

Ring A is a 5-7 membered aliphatic ring and may optionally be mono- or di-substituted by optionally substituted C1-6 alkyl or C1-6 alkoxy, each substituent being independently selected from halogen, C1-6 alkyl or an oxo group; $x_1$ and $x_2$ are independently selected from N, O and C, where a ring substituent on ring A is an oxo group this is preferably adjacent a ring nitrogen atom;

Ring B is a monocyclic or bicyclic alkyl, aryl, aralkyl, heteroaryl or heteroaralkyl ring comprising up to 12 ring atoms and containing no, one or up to three heteroatoms independently chosen from N, O, and S; alternatively ring A may be omitted;

each $R_3$ is independently selected from hydrogen, halogen, $-NO_2$, -CN, $-CF_3$, -OH, $-NH_2$, C1-6 alkyl, -S-C1-6 alkyl, -SO-C1-6 alkyl, $-SO_2$-C1-6 alkyl, $-SO_2-NH_2$, C1-6 alkoxy, up to C10 aryloxy, and COOR wherein R is hydrogen or C1-6 alkyl, n is 1-5;

L is -CO- or a direct bond;

$R_1$ and $R_2$ are independently selected from hydrogen, halogen, or C1-6 alkyl;

Y is nitrogen or sulphur;

Q is selected from -CO-, $-SO_2-$, $-CR_6R_7-$, wherein $R_6$ and $R_7$ independently selected from H, -OH-, C1-6 alkyl, C5-7 cycloalkyl, up to C10 aryl, up to C10 heteroaryl, up to C12 aralkyl, or up to C12 heteroarylalkyl;

$R_4$ is H, C1-6 alkyl, C5-7 cycloalkyl, up to C10 aryl, up to C10 heteroaryl, up to C12 aralkyl, or up to C12 heteroarylalkyl, all optionally substituted by up to three groups independently selected from $-NO_2$, -OH, $-CF_3$, $-NH_2$, $-CO-NH_2$, -COOH, halogen, C1-4 alkyl, C1-4 alkyloxy, carboxy (C1-4) alkyl, or up to C6 cycloalkyl;

$R_5$ is H, C1-6 alkyl, or together with $R_4$ forms a carbocyclic or heterocyclic 5, 6 or 7 membered ring, the latter containing at least one or up to three heteroatoms selected from N, O, and S and may optionally substituted by one or up to four oxo groups;

Z is a 5, 6 or 7 membered alkyl, aryl, heteroalkyl, or heteroaryl ring comprising one or up to three of O, S and N and may optionally substituted by one or up to four oxo groups; alternatively Z is a nitrogen optionally substituted by hydrogen, C1-4 alkyl, C5-7 cycloalkyl, up to C10 aryl, up to C10 heteroaryl, up to C12 aralkyl, or up to C12 heteroarylalkyl, -CO-C1-6 alkyl, -CO-C1-6 aryl, -CO-C1-6 heteroaryl, $-SO_2$-C1-6 alkyl, $-SO_2$-C1-6 aryl, $-SO_2$-C1-6 heteroalkyl, $-SO_2$-C1-6 heteroaryl, $-SO_2$-CH2-C1-6 aryl, $-SO_2$-CH2-C1-6 heteroalkyl, $-SO_2$-CH2-C1-6 heteroaryl, heteroatoms selected from N, O or S, all rings optionally substituted by up to three groups independently selected from hydrogen, halogen, -OH, -COOH, $-CH_2$-COOH, -CN, $-CF_3$, C1-6 alkyl, or C1-6 alkoxy; alternatively Z is a hydrogen.

Any alkyl groups outlined above may be straight chain or branched. It will be appreciated that the particular substitutients and number of substituents on rings A and B are selected so as to avoid sterically undesirable combinations. Depending on substituents, the compounds of formula (**I**) may exist in geometric, optical and other isomeric forms and this invention embraces any of these isomers or enantiomers.

A second aspect of this invention relates to methods for preparing compounds of formula (**I**).

A third aspect of this invention relates to pharmaceutical compositions containing a therapeutically effective amount of a compound of formula (**I**) or a pharmaceutically acceptable salt thereof admixed with at least one pharmaceutically acceptable excipient.

A fourth aspect of this invention relates to methods for treating mammals having a disease state alleviated by the inhibition of matrix metalloproteinases or by the inhibition of ADAMs, by administering an effective amount of a compound of formula (**I**), or a pharmaceutical composition thereof, to the mammal. Such disease states include arthritic diseases (rheumatoid arthritis and osteoarthritis), bone resorptive diseases (such as osteoporosis), the enhanced collagen destruction associated with diabetes, the enhanced collagen destruction associated with ultraviotet-B irradiation, chronic obstructive pulmonary disease, cerebral hemorrhaging associated with stroke, periodontal disease, corneal or gastric ulceration, ulceration of the skin, tumor invasion and metastasis.

Illustrative examples of compounds according to preferred embodiments of the present invention include, but are not limited to:

1. (2R)-N-[5-(4-Cyanophenyl)-6H-1,3,4-thiadiazin-2-yl]-2-[(phenylsulfonyl)amino]-propanamide
2. (2R)-N-[5-(4-Chlorophenyl)-6H-1,3,4-thiadiazin-2-yl]-3-methyl-2-[(phenylsulfonyl)amino]butanamide
3. (2S)-N-[5-(4-Cyanophenyl)-6H-1,3,4-thiadiazin-2-yl]-2-[(phenylsulfonyl)amino]-propanamide
4. (2S)-2-[(Phenylsulfonyl)amino]-N-{5-[4-(trifluormethyl)phenyl]-6H-1,3,4-thiadiazin-2-yl}propanamide
5. (2S)-2-[(Benzylsulfonyl)amino]-N-[5-(4-chlorophenyl)-6H-1,3,4-thiadiazin-2-yl]propanamide

6. (2S)-N-[5-(4-Bromophenyl)-6H-1,3,4-thiadiazin-2-yl]-2-[(phenylsulfonyl)amino]-propanamide
7. (2R)-N-[5-(4-Bromophenyl)-6H-1,3,4-thiadiazin-2-yl]-2-[(phenylsulfonyl)amino]-propanamide
8. (2S)-N-[5-(1-Adamantyl)-6H-1,3,4-thiadiazin-2-yl]-2-[(phenylsulfonyl)amino]-propanamide
9. (2S)-N-[5-(4-Nitrophenyl)-6H-1,3,4-thiadiazin-2-yl]-2-[(phenylsulfonyl)amino]-propanamide
10. (2S)-N-[5-(5-Chloro-2-thienyl)-6H-1,3,4-thiadiazin-2-yl]-2-[(phenylsulfonyl)-amino]propanamide
11. (2S)-N-[5-(4-Chlorophenyl)-6H-1,3,4-thiadiazin-2-yl]-2-[(phenylsulfonyl)amino]-propanamide
12. (2S)-N-[5-(4-Methoxyphenyl)-6H-1,3,4-thiadiazin-2-yl]-2-[(phenylsulfonyl)-amino]propanamide
13. (2S)-N-[5-(4-Fluorophenyl)-6H-1,3,4-thiadiazin-2-yl]-2-[(phenylsulfonyl)amino]-propanamide
14. (2R)-N-[5-(4-Fluorophenyl)-6H-1,3,4-thiadiazin-2-yl]-2-[(phenylsulfonyl)amino]-propanamide
15. (2R)-N-[5-(4-Methylphenyl)-6H-1,3,4-thiadiazin-2-yl]-2-[(phenylsulfonyl)-amino]propanamide
16. 2-{[(4-Methoxyphenyl)sulfonyl]amino}-N-[5-(2,3,4,5,6-pentafluorophenyl)-6H-1,3,4-thiadiazin-2-yl]acetamide
17. (2S)-2-{[(4-Methoxyphenyl)sulfonyl]amino}-N-[5-(4-methylphenyl)-6H-1,3,4-thiadiazin-2-yl]propanamide
18. 2-{[(4-tert-Butylphenyl)sulfonyl]amino}-N-[5-(2,4-dichlorophenyl)-6H-1,3,4-thiadiazin-2-yl]acetamide
19. N-[5-(2,4-Dichlorophenyl)-6H-1,3,4-thiadiazin-2-yl]-2-{[(4-methoxyphenyl)-sulfonyl]amino}acetamide
20. N-[5-(4-Chlorophenyl)-6H-1,3,4-thiadiazin-2-yl]-2-methyl-2-[(phenylsulfonyl)-amino]propanamide
21. (2R)-N-[5-(4-Chlorophenyl)-6H-1,3,4-thiadiazin-2-yl]-2-[(phenylsulfonyl)amino]-propanamide
22. (2S)-N-[5-(4-Chlorophenyl)-6H-1,3,4-thiadiazin-2-yl]-3-methyl-2-[(phenylsulfonyl)amino]butanamide
23. N-[5-(4-Chlorophenyl)-6H-1,3,4-thiadiazin-2-yl]-2-(2,5-dioxo-4-imidazolidinyl)-acetamide
24. N-[5-(4-Bromophenyl)-6H-1,3,4-thiadiazin-2-yl]-2-(2,5-dioxo-4-imidazolidinyl)-acetamide
25. N-[5-(4-Cyclohexylphenyl)-6H-1,3,4-thiadiazin-2-yl]-2-(2,5-dioxo-4-imidazolidinyl)acetamide
26. 2-(2,5-Dioxo-4-imidazolidinyl)-N-{5-[4-(1-pyrrolidinyl)phenyl]-6H-1,3,4-thiadiazin-2-yl}acetamide
27. (2S)-N-[5-(4-Chlorophenyl)-6H-1,3,4-thiadiazin-2yl-]-2-[(2-thienylsulfonyl)-amino]propanamide
28. (2S)-N-[5-(4-Chlorophenyl)-6H-1,3,4-thiadiazin-2yl-]-2-[(4-morpholinylsulfonyl)amino]propanamide
29. (2S)-N-[5-(4-Chlorophenyl)-6H-1,3,4-thiadiazin-2-yl]-2-[(3-pyridylsulfonyl)-amino]propanamide
30. (4S)-N-[5-(4-Chlorophenyl)-6H-1,3,4-thiadiazin-2-yl]-2,6-dioxohexahydro-4-pyrimidinecarboxamide
31. N-Allyl-5-(4-chlorophenyl)-6H-1,3,4-thiadiazin-2-amine hydrobromide

DETAILED DESCRIPTION OF THE INVENTION

As previously outlined the compounds of the invention are inhibitors of metalloproteinases. Recent efforts by a number of laboratories have provided several classes of MMP inhibitors which have been extensively reviewed[15]. In general, each class contains a zinc ligand attached to a small peptide fragment which is capable of binding to specificity pockets of the MMP enzymes, usually on the primed side. In general, the use of hydroxamates as the zinc ligand provides for the most effective MMP inhibitors. However, hydroxamates are often found to be biologically labile which has prompted additional efforts toward the discovery of new chelating groups suitable for use in MMP inhibitor templates. To explore this issue further, we undertook a broad screening effort using the catalytic domain of MMP-8 as the screening enzyme. From this, a number of chiral thiadiazine amines were identified which were weak (>40 μM) inhibitors of cdMMP-8. After elaboration of this compound series, improvement of enzyme potency, determination of structure-activity-relationships (SAR) and determination of MMP-specificity, we have found, that thiadiazines of formula (**I**) potentially are novel, nonpeptidic alternatives to peptide-mimetic hydroxamate MMP inhibitors. Under the assumption that the exo-cyclic nitrogen chelates to the catalytic zinc, with the thiadiazine-heterocycle and its position-5 residue generally located toward the unprimed side, several amide analogues were synthesized to improve primed side binding affinity. However the most effective analogues ($K_i$-values around 50 nM) utilized an N-aryl-sulphonyl substituted D-alanine as the acid component and an p-chloro- or p-bromo-substituted arylthiadiazine-amine as the amine component. Further optimization leads to the pharmaceutically interesting achiral compound N-[5-(4-chlorophenyl)-6H-1,3,4-thiadiazin-2-yl]-2-methyl-2-[(phenylsulfonyl)amino]propanamide which displays $K_i$-values around 100 nM. The structure-activity-relationships for the compounds described within this invention are rationalized through the consideration of the X-ray structure recently attained for N-allyl-5-(4-chlorophenyl)-6H-1,3,4-thiadiazin-2-amine hydrobromide complexed to cdMMP-8[16]. Selected compounds of this invention were further assayed *in vitro* for the inhibition of the catalytic domain of ADAM-9 and show remarkable selectivity. These results suggest that thiadiazines can provide selective inhibitors of MMPs or ADAMs with a novel binding mode and increased oral bioavailablity because of their non-peptidic nature.

Dispite of this, the compounds of the present invention are also useful scientific research tools for studying functions and mechanisms of action of matrix metalloproteinases or ADAMs in both *in vivo* and *in vitro* systems. For example the present compounds can be used to modulate MMP action, thereby allowing the researcher to observe the effects of reduced MMP activity in the experimental biological system under study.

The following thiadiazines are preferred in addition to the compounds mentioned in the examples and compounds derived by combination of all meanings of substitutes mentioned in the claims:

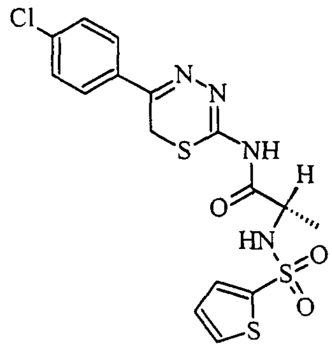

| | | |
|---|---|---|
| $K_i$ [nM] | MMP-1 | 1044 |
| | cdMMP-2 | 547 |
| | cdMMP-8 | 64 |
| | MMP-9 | 798 |
| | cdMMP-12 | 361 |
| | cdMMP-13 | 3810 |
| | cdMMP-14 | 255 |

(2S)-N-[5-(4-Chlorophenyl)-6H-1,3,4-thiadiazin-2yl-]-2-[(2-thienylsulfonyl)amino]propanamide

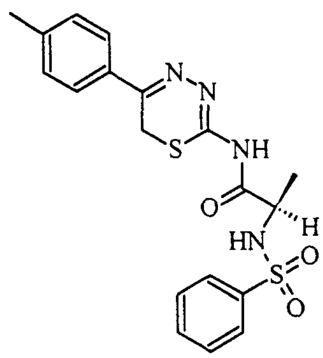

| | | |
|---|---|---|
| $K_i$ [nM] | MMP-1 | 457 |
| | cdMMP-2 | 444 |
| | cdMMP-8 | 220 |
| | MMP-9 | 213 |
| | cdMMP-12 | 96 |
| | cdMMP-13 | 315 |
| | cdMMP-14 | 288 |
| | ΔTM-MMP-14 | 152 |

(2R)-N-[5-(4-Methylphenyl)-6H-1,3,4-thiadiazin-2-yl]-2-[(phenylsulfonyl)amino]propanamide

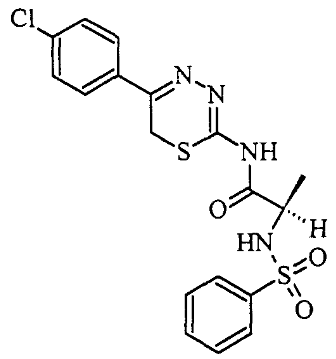

| | | |
|---|---|---|
| $K_i$ [nM] | MMP-1 | 210 |
| | cdMMP-2 | 500 |
| | cdMMP-8 | 146 |
| | MMP-9 | 84 |
| | cdMMP-12 | 321 |
| | cdMMP-13 | 136 |
| | cdMMP-14 | 50 |
| | ΔTM-MMP-14 | 207 |

(2R)-N-[5-(4-Chlorophenyl)-6H-1,3,4-thiadiazin-2-yl]-2-[(phenylsulfonyl)amino]propanamide

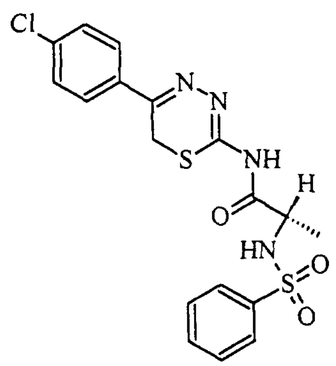

| | | |
|---|---|---|
| $K_i$ [nM] | MMP-1 | 652 |
| | cdMMP-2 | 940 |
| | cdMMP-8 | 734 |
| | MMP-9 | 53 |
| | cdMMP-12 | 515 |
| | cdMMP-13 | 183 |
| | cdMMP-14 | 100 |
| | ΔTM-MMP-14 | 213 |

(2S)-N-[5-(4-Chlorophenyl)-6H-1,3,4-thiadiazin-2-yl]-2-[(phenylsulfonyl)amino]propanamide

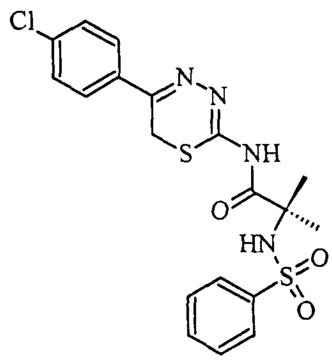

| | | |
|---|---|---|
| $K_i$ [nM] | MMP-1 | 302 |
| | cdMMP-2 | 79 |
| | cdMMP-8 | 481 |
| | MMP-9 | 95 |
| | cdMMP-12 | 198 |
| | cdMMP-13 | 177 |
| | cdMMP-14 | 146 |
| | ΔTM-MMP-14 | 101 |

N-[5-(4-Chlorophenyl)-6H-1,3,4-thiadiazin-2-yl]-2-methyl-2-[(phenylsulfonyl)amino]propanamide

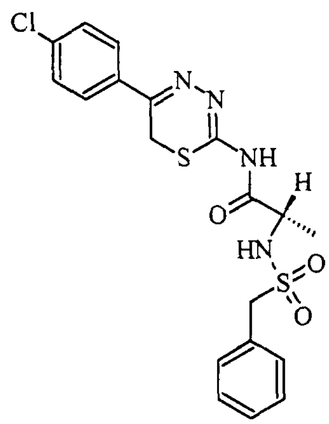

| | | |
|---|---|---|
| $K_i$ [nM] | MMP-1 | 359 |
| | cdMMP-2 | 98 |
| | cdMMP-8 | 201 |
| | MMP-9 | 50 |
| | cdMMP-12 | 483 |
| | cdMMP-13 | 119 |
| | cdMMP-14 | 190 |
| | ΔTM-MMP-14 | 181 |

(2S)-2-[(Benzylsulfonyl)amino]-N-[5-(4-chlorophenyl)-6H-1,3,4-thiadiazin-2-yl]propanamide

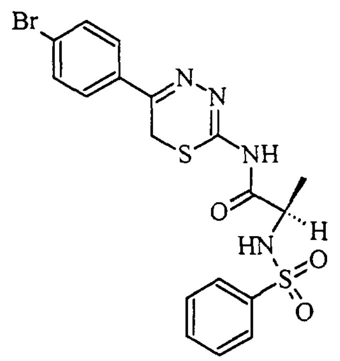

| | | |
|---|---|---|
| $K_i$ [nM] | MMP-1 | 432 |
| | cdMMP-2 | 145 |
| | cdMMP-8 | 212 |
| | MMP-9 | 38 |
| | cdMMP-12 | 339 |
| | cdMMP-13 | 131 |
| | cdMMP-14 | 197 |
| | ΔTM-MMP-14 | 179 |

(2R)-N-[5-(4-Bromophenyl)-6H-1,3,4-thiadiazin-2-yl]-2-[(phenylsulfonyl)amino]propanamide

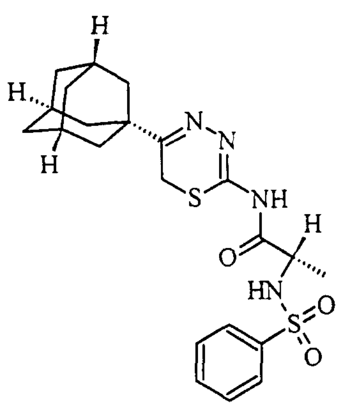

| | | |
|---|---|---|
| $K_i$ [nM] | cdMMP-2 | 8%[a] @ 15 μM |
| | cdMMP-8 | 32%[a] @ 15 μM |
| | MMP-9 | 21%[a] @ 15 μM |
| | cdMMP-12 | 405 |
| | cdMMP-13 | 41%[a] @ 15 μM |
| | cdMMP-14 | 3080 |

[a] percent inhibition

(2S)-N-[5-(1-Adamantyl)-6H-1,3,4-thiadiazin-2-yl]-2-[(phenylsulfonyl)amino]propanamide

PHARMACEUTICAL COMPOSITIONS

The pharmaceutical compositions of this invention may be prepared by combining the compounds of formula (**I**) of this invention with a solid or liquid pharmaceutically acceptable carrier, and optionally, with pharmaceutically acceptable adjuvants and excipients employing standard and conventional techniques.

The pharmaceutical compositions according to the invention are those suitable for enteral, such as oral or rectal, transdermal, and parenteral administration to mammals, including man, to inhibit matrix metalloproteinases, and for the treatment of disorders responsive thereto, comprising an effective amount of a pharmacologically active compound of formula (**I**), alone or in combination, with one or more pharmaceutically acceptable carriers.

Preferred are tablets and gelatin capsules comprising the active ingredient together with diluents, e.g. lactose,

dextrose, sucrose, mannitol, sorbitol, and cellulose; lubricants, e.g. silica, talcum, stearic acid, its magnesium or calcium salt and/or polyethyleneglycol; for tablets also binders, e.g. magnesium aluminium silicate, starch paste, gelatin, tragacanth, methylcellulose, sodium carboxymethylcellulose and or polyvinylpyrrolidone; if desired disintegrants, e.g. starches, agar, alginic acid or its sodium salt, or effervescent mixtures; and/or absorbants, colorants, flavors and sweeteners. Suppositories are advantageously prepared from fatty emulsions or suspensions.

Pharmaceutical compositions for parenteral administration will generally contain a pharmaceutically acceptable amount of the compounds according to formula (**I**) as a soluble salt dissolved in a pharmaceutically acceptable liquid carrier such as, for example, water-for-injection and a suitably buffered isotonic solution having a pH of about 3.5 to 6.0. Pharmaceutically acceptable salts are well known in the art. They are described in detail for example by S. M. Berge et al[17]. Pharmaceutically acceptable salts of the acidic compounds of the invention are salts formed with bases, namely cationic salts such as alkali and alkaline earth metal salts, such as sodium, lithium, potassium, calcium, magnesium, as well as ammonium salts, such as ammonium, trimethylammonium, diethylammonium, and tris-(hydroxymethyl)-methyl-ammonium salts. Similarly acid addition salts, such as of mineral acids, organic carboxylic and organic sulphonic acids, e.g. hydrochloric acid, methanesulphonic acid, maleic acid, are also possible provided a basic group, such as amino, morpholino or pyridyl, constitutes part of the structure. Said compositions may be sterilized and/or contain adjuvants, such as preserving, stabilizing, wetting or emulsifying agents, solution promotors, salts for regulating the osmotic pressure and/or buffers. Suitable buffering agents include, for example, trisodium orthophosphate, sodium bicarbonate, sodium citrate, N-methylglucamine, L(+)-lysine and L(+)-arginine, to name a few. In addition, they may also contain other therapeutically valuable substances. Said compositions are prepared according to conventional mixing, granulating or coating methods, respectively, and contain about 0.1 to 75%, preferably about 1 to 50%, of the active ingredient.

Suitable formulations for transdermal application include an effective amount of a compound of formula (**I**) with an carrier. Advantageous carriers include absorbable pharmacologically acceptable solvents to assist passage through the skin of the host. Characteristically, transdermal devices are in the form of a bandage comprising a backing member, a reservoir containing the compound optionally with carriers, optionally a rate controlling barrier to deliver the compound of the skin of the host at a controlled and predetermined rate over a prolonged time, and means to secure the device to the skin.

Solar UV radiation damages human skin, affecting skin tone and resiliency and leading to premature aging (photoaging), the symtoms of which include leathery texture, wrinkles, mottled pigmentation, laxity and sallowness. This photoaging results largely from UV induction of matix metalloproteinases that degrade skin collagen. Pretreatment of human skin with all-*trans* retinoic acid (tRA) inhibits UV induction of matix metalloproteinases, suggesting that tRA can protect against UV-induced collagen destruction and may therefore be able to lessen the effects of photoaging[18]. According to this, the UV-induced collagen destruction may be prevented at an more effective level by the topical application of a compound of formula (**I**) in conjunction with all-*trans* retinoic acid to the skin prior to its exposure to UV radiation. Suitable formulations for topical application, e.g. to the skin and eyes, are preferably aqueous solutions, ointments, creams or gels well-known in the art. The pharmaceutical formulations contain an effective matrix metalloproteinase inhibiting amount of a compound of formula (**I**) as defined above either alone, or in combination with another therapeutic agent, e.g. all-trans retinoic acid, an antiinflammatory agent with cyclooxygenase inhibiting activity such as diciofenac sodium, other antirheumatic agents such as methotrexate, or antitumor agents such as bleomycin each at an effective therapeutic dose as reported in the art. Other therapeutic agents are well-known in the art.

In conjunction with another active ingredient, a compound of formula (**I**) may be administered either simultaneously, before or after the other active ingredient, either separately by the same, by a different route of administration or together in the same pharmaceutical formulation. The dosage of active compound administered is dependent on the species of warm-blooded mammal, the body weight, age and individual condition, and on the form of administration. A unit dosage for oral administration to a mammal of about 50 to 70 kg may contain between about 10 and 1000 mg, advantageously between about 25 and 250 mg of the active ingredient.

## IN VITRO METALLOPROTEINASE INHIBITION

### Abbreviations

Abbreviations which have been used in the description that follows are: APMA for p-aminophenylmercuric acetate, MCA for (7-methoxycoumarin-4-yl)acetyl-, MRB for Microfluorometric Reaction Buffer, HEPES for N-[2-hydroxyethyl]piperazine-N'-[2-ethanesulphonic acid], PEG 8000 for polyethyleneglycol av. mol. wt.: 8000, PMNL for polymorphonuclear neutrophile leucocytes, DMSO for dimethylsulfoxide, MMP for matrix metalloproteinases, M for molar, cd for catalytic domain, ΔTM for without transmembrane moiety, TKI for Trypsin-Kallikrein-Inhibitor, and [S] for substrate concentration,

MMPs

The tested MMP enzymes were prepared more specifically in the laboratories of the applicants, with the exception of MMP-1. MMP-1 from human rheumatoid synovial fibroblasts was purchased from Calbiochem-Novabiochem Corporation, LaJolla. Preparation of the recombinant catalytic domain of human Gelatinase A (cdMMP-2): The catalytic domain of Pro-Gelatinase A was prepared using an *E. coli* expression system according to the method described by Kröger et al[19]. The proenzyme was activated with 0.5 mM APMA at 37°C prior to use in the assay. Preparation of the recombinant catalytic domain of human neutrophile Collagenase (cdMMP-$8^{Met80}$): The enzyme was expressed in *E. coli* as an active variant by the method of Kleine et al[20]. Preparation of PMNL-Gelatinase (MMP-9): Latent PMNL-pro-Gelatinase was prepared from human plasma buffy coat as described by Tschesche et al[21]. PMNL-pro-Gelatinase was activated prior to use by incubation with Trypsin at 37°C for 10 min. Inactivation of Trypsin was accomplished with TKI. Preparation of the recombinant catalytic domain of human Macrophage Elastase (cdMMP-12): The catalytic domain of MMP-12 was expressed in *E. coli*. The overexpressed protein was isolated as inclusion bodies and the renatured protein was purified by affinity chromatography with a hydroxamate inhibitor coupled column as described[22]. Preparation of the recombinant catalytic domain of human Collagenase-3 (cdMMP-13): Human pro-cdMMP-13 was prepared using an *E. coli* expression system according to the method described[23]. The isolated pro-cdMMP-13 was activated prior to use by incubation with 5 mM $HgCl_2$ for 2 h at 37°C. Preparation of the recombinant catalytic domain of Membrane-Type-1 MMP (cdMMP-14): The catalytic domain of MMP-14 was expressed in *E. coli* and was activated by autocatalysis[24]. Preparation of the recombinant human ectodomain of Membrane-Type-1 MMP (ΔTM-MMP-14): The ectodomain of MMP-14 was expressed in *Pichia Pastoris* by the method of Roderfeld et al. and activated by yeast proteases (Furin-like-proteases) during maturation[25].

Determination of MMP inhibition

The enzymatic activity was measured using a modified version of a resonance energy transfer fluorogenic assay as described[26]. Progress curves were monitored by following the increase in fluorescence at 393 nm ($\lambda_{ex}$ = 328 nm), induced by the cleavage of the (7-methoxycoumarin-4-yl)acetyl-Pro-Leu-Gly-Leu-(3-[2,4-dinitro-phenyl]-L-2,3-diamino-propionyl)-Ala-Arg-$NH_2$ (Mca-Pro-Leu-Gly-Leu-Dpa-Ala-Arg-$NH_2$) fluorogenic substrate by MMPs. The fluorescent MCA-group is quenched by resonance energy transfer to the 2,4-dinitrophenyl group. Matrix metalloproteinases cleave this substrate at the Gly-Leu bond. Cleavage results in the loss of energy transfer and a large increase in fluorescence of the MCA group. This substrate is commercially available from Bachem, Switzerland.

Enzyme inhibition assays were carried out in MRB which consisted of 50 mM HEPES/NaOH, pH 7.0, 10 mM $CaCl_2$ and 0,02% (w/v) PEG 8000 at 25°C. MMP microfluorometric profiling assay was done in white U-bottomed 96-well plates (Microfluor 1 White, Dynex, USA) with a final substrate concentration of 6 μM MCA-peptide, approximately 0.3 to 5 nM MMP with variable inhibitor concentrations and 2% DMSO vehicle. From a 1.5 mM stock (100% DMSO) the inhibitors were serially diluted with MRB to 15, 10, 5, 3, 1, 0.6, 0.3, 0.1, 0.05, 0.01 and 0.005 μM final assay concentration. Column 1, rows A through H, contained only DMSO for the "enzyme-only" wells in the assay. After preincubation for 30 min at 25°C the reaction was started by addition of substrate and the plate was read on a SpectraFluor Plus (Tecan, Germany) plate reader with excitation at 330 nm and emission at 405 nm with the gain set to 150. Each measurement was done in triplicate to ensure statistically significant results. The experiment was further controlled for background fluorescence of the substrate, for fluorescence of fully cleaved substrate and for fluorescence quenching or augmentation from solutions containing the test compounds.

IC50 and Ki determination in microfluorometric assay

Datapoints from eight different MMPs generated on the SpectraFluor Plus were directly visualized on a master Excel spreadsheet. The response of inhibition was determinated for each inhibitor concentration by comparing the amount of hydrolysis (fluorescence units generated over 30 minutes of hydrolysis) of wells containing compound with the "enzyme-only" wells in column 1. With the program GraFit (Erithacus Software Limited) a 4 parameter logistic fit to the dose-response data was used to calculate $IC_{50}$ values for each compound.

For each MMP, initial rate measurements in the absence of inhibitor were made for eight different substrate concentrations. From these data, $K_m$ values were determined by nonlinear fit using the program GraFit. The $K_m$ values determined for MMP-1, cdMMP-2, cdMMP-8, MMP-9, cdMMP-12, cdMMP-13, cdMMP-14 and ΔTM-MMP-14 were 40.3, 9.1, 5.9, 1.8, 27.3, 7.5, 6.8, and 7.5 μM, respectively.

Assuming competitive inhibition $K_i$ values were calculated automatically for each enzyme tested based upon the equation previously described by Cheng and Prusoff[27]:

$$K_i = (K_m \times IC_{50})/(K_m + [S])$$

Where [S] = 6 μM. The results of the obtained tests are presented previously with the preferred examples, and below in Table 1.

Table 1

| Example | MMP-1 $IC_{50}$ (μM) | cdMMP-2 $IC_{50}$ (μM) | cdMMP-8 $IC_{50}$ (μM) | MMP-9 $IC_{50}$ (μM) | cdMMP-12 $IC_{50}$ (μM) | cdMMP-13 $IC_{50}$ (μM) | cdMMP-14 $IC_{50}$ (μM) | ΔTM-MMP-14 $IC_{50}$ (μM) |
|---|---|---|---|---|---|---|---|---|
| 1 | 0.451 | 0.353 | 0.468 | 0.232 | 0.444 | 0.368 | 0.346 | 0.610 |
| 2 | 0.629 | 1.41 | 8.63 | 40%a)@ 15 | 0.426 | > 15 | 1.11 | 0.593 |
| 3 | 0.449 | 0.390 | 0.440 | 0.329 | 0.447 | 0.387 | 0.459 | 0.709 |
| 4 | 0.490 | 25%a)@ 15 | 0.515 | 1.04 | 0.367 | 1.03 | 1.10 | 0.708 |
| 6 | n.d. | 0.455 | 0.231 | 0.686 | 0.533 | 0.658 | 0.637 | n.d. |
| 9 | 0.294 | 0.495 | 0.337 | 0.552 | 0.342 | 0.449 | 0.556 | n.d. |
| 10 | 0.520 | 3.10 | 0.371 | 0.722 | 0.298 | 0.853 | 0.605 | 0.357 |
| 12 | 0.663 | 0.857 | 0.354 | 1.40 | 0.412 | 2.31 | 1.12 | n.d. |
| 13 | 0.500 | 0.743 | 0.383 | 0.684 | 0.338 | 1.17 | 0.839 | n.d. |
| 14 | 0.725 | 0.572 | 0.702 | 0.216 | 0.425 | 0.537 | 0.727 | 0.596 |
| 16 | n.d. | 3.93 | 0.527 | 2.57 | 0.920 | 1.09 | 1.76 | n.d. |
| 17 | n.d. | 0.675 | 0.599 | 0.689 | 0.436 | 1.11 | 0.707 | n.d. |
| 18 | 0.424 | 2.43 | 0.492 | 2.60 | 0.657 | 1.41 | 0.780 | 0.887 |
| 19 | n.d. | 1.52 | 0.306 | 0.661 | 0.259 | 0.605 | 0.700 | n.d. |
| 22 | n.d. | 1.46 | 1.58 | 0.851 | 0.463 | >15 | 1.40 | n.d. |
| 23 | n.d. | 67%a)@ 15 | 0.648 | 40%a)@ 15 | 0.380 | 34%a)@ 15 | 52%a)@ 15 | n.d. |
| 24 | n.d. | 0.864 | 0.578 | 45%a)@ 15 | 0.297 | 39%a)@ 15 | 1.07 | n.d. |
| 25 | 67%a)@ 15 | 53%a)@ 15 | 56%a)@ 15 | 49%a)@ 15 | 1.42 | 67%a)@ 15 | 66%a)@ 15 | n.d. |
| 26 | n.d. | 0.890 | 1.17 | 0.517 | 0.217 | 36%a)@ 15 | 1.10 | 63%a)@ 15 |
| 28 | 0.537 | 0.662 | 0.296 | 1.20 | 0.330 | 0.735 | 0.471 | n.d. |
| 29 | 0.507 | 0.354 | 0.377 | 0.257 | 0.403 | 0.361 | 0.443 | 0.430 |
| 30 | 0.560 | 0.693 | 0.692 | 0.780 | 0.654 | 0.947 | 0.477 | 0.692 |
| 31 | n.d. | 3.31 | 51.90 | 0.783 | > 15 | > 15 | 2.70 | n.d. |

a) = percent inhibition; n.d. = not detected

INHIBITION OF THE ADAMALYSIN FAMILY INCLUDING ADAM-9 AS A REPRESENTATIVE OF ITS CLASS

ADAMs

The tested recombinant catalytic domain of ADAM-9 was prepared in the laboratories of the applicants by the method of Schwettmann et al[28]. The enzyme was expressed in *Pichia Pastoris* and activated by yeast proteases

during maturation.

Test procedure for measuring cdADAM-9 inhibition

Quartz cuvettes received a solution composed of 7 µL cdADAM-9 (final concentration 0.2 nM), 2 mL Tris buffer, pH 7.5 (20 mM Tris/HCl, 500 mM NaCl, 5 mM $CaCl_2$ and 0.5 mM $ZnCl_2$) containing 14 µL of compound **31** solution in $H_2O$ (final concentration: 20 µM) and were incubated for 30 minutes at 37°C. The reaction was initiated by addition of a fluorogenic peptide substrate for TNF-α converting enzyme (Mca-Pro-Leu-Ala-Gln-Ala-Val-Dap(Dnp)-Arg-Ser-Ser-Ser-Arg-$NH_2$, final concentration: 2 µM) to each cuvette. This substrate is commercially available from Bachem, Switzerland.

Initial rates of cleavage were monitored by measuring the rate of increase in fluorescence at 393 nm ($\lambda_{ex}$ = 328 nm) over 60 min with a LS50B fluorimeter (Perkin Elmer, Germany). The increase in fluorescence over time was plotted as a linear line. The slope of the line was calculated and represents the reaction rate. The rate of reaction in the presence of test compound was compared to that in the absence of test compound.

PREPARATION OF COMPOUNDS OF FORMULA (I)

Abbreviations

Abbreviations which have been used in the descriptions of the schemes and the examples that follows are: EDCI for N-(3-dimethyl-aminopropyl)-N'-ethyl-carbodiimide hydrochloride, NMM for 4-methylmorpholine, HOBT for 1-hydroxy-benzotriazole, DMF for dimethylformamide, EEDQ for N-ethoxycarbonyl-2-ethoxy-1,2-dihydroquinoline, EtOH for ethanol, MeOH for methanol, TLC for Thin-Layer Chromatography, mp for melting point, TMS for tetramethylsilane, DCI for Direct Chemical Ionization, MALDI for Matrix Assisted Laser Desorption/Ionisation, TOF for Time Of Flight, DHB for 2,5-dihydroxybenzoic acid, dec for decomposition, and DEI for Direct Electron Impact.

Chemistry

The compounds of the invention may be prepared by the use of known chemical reactions and procedures. Nevertheless, the following general preparative methods are presented to aid the reader in synthesizing the inhibitors, with more detailed particular examples being presented below in the experimental section describing the working examples. The preferred process involves the acylation of a substituted 1,3,4-thiadiazine amine hydrohalide with an appropriately substituted sulphonamide of an amino acid derivative or an substituted carboxylic acid derivative to provide the corresponding 1,3,4-thiadiazine amide according to the following reaction scheme:

Scheme 1

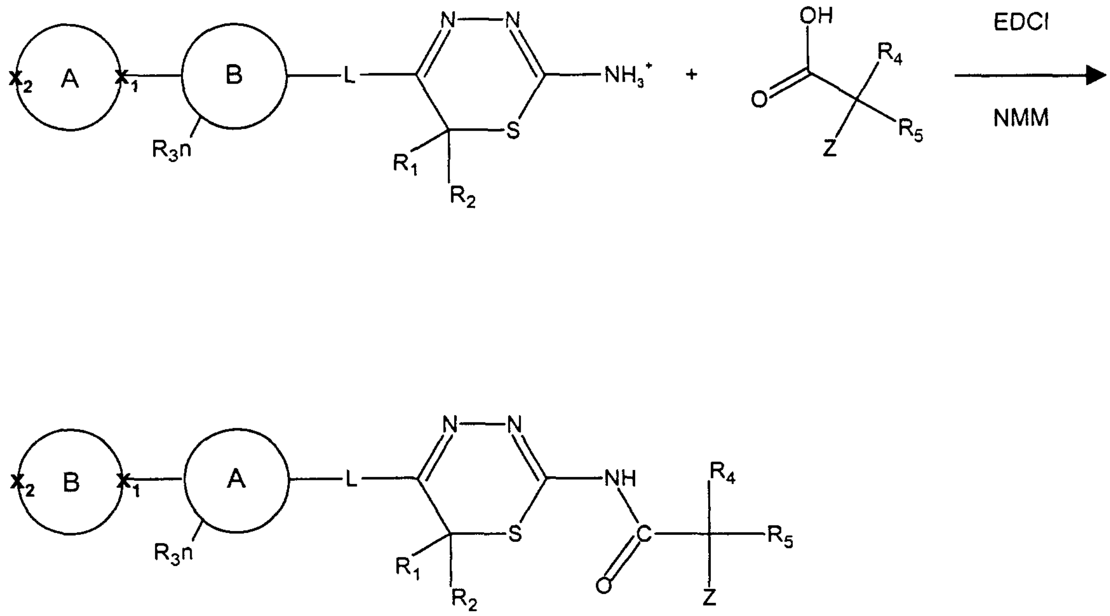


wherein $R_1$, $R_2$, $R_{3n}$, $R_4$, $R_5$, L, $X_1$, $X_2$, A, B and Z are as defined above. The preferred synthetic procedure involves the direct coupling of the carboxylic acid derivative with a thiadiazine amine hydrohalide. Such coupling reaction necissitates the use of a coupling reagent, for example any of the type of coupling reagents commonly employed in the synthesis of peptides. Examples of such coupling reagents include the carbodiimides such as N,N'-dicyclohexylcarbodiimide, N,N'-diisopropylcarbodiimide, or N-(3-dimethylaminopropyl)-N'-ethylcarbodiimidehydro-chloride, the imidazoles such as carbonyldiimidazole; as well as reagents such as EEDQ. The preferred coupling reagent used in this invention is EDCI. The direct coupling of a carboxylic acid and a thiadiazine amine hydrohalide is carried out by combining about equimolar quantities of the starting materials in the presence of an equimolar quantity of HOBT, an slight excess of NMM, and in the presence of an equimolar quantity or slight excess of the coupling reagent. The reaction is carried out in an unreactive organic solvent such as DMF, and usually is complete within about fourty-eight hours when conducted at a temperature of 0°C to about 5°C. The thiadiazine amide is readily isolated and purified by standard procedures.

The preferred 1,3,4-thiadiazine amine hydrohalides or compounds of formula (**I**) are prepared by reacting a substituted phenacyl halide of the formula (**II**)

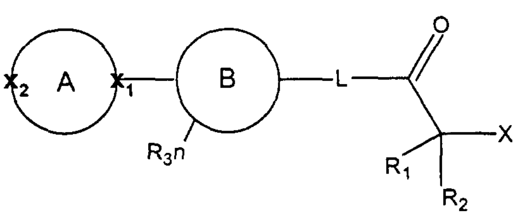

(**II**)

wherein $R_1$, $R_2$, L, $R_{3n}$, $X_1$, $X_2$, A and B are as defined above, and X is Cl or Br, preferably Br, with thiosemicarbazide, thiosemicarbazide hydrochloride or with a 4-substituted thiosemicarbazide of the formula (**III**)

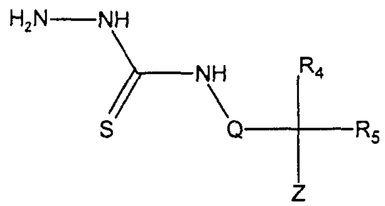

(III)

wherein Q is $-CR_6R_7-$, $R_4$, $R_5$ and Z are as hereinbefore defined. The reaction is generally conducted in the presence of a solvent, e.g. a lower alkanol, such as, methanol, ethanol, isopropanol, n-propanol, n-butanol and the like, preferably ethanol. In the case of substituted phenacylbromides the reaction includes two steps. In the first step the stirred reaction mixture is allowed to warm up from 0°C to about 18°C within twelve hours to yield the ring-opened intermediate. The intermediate is generally worked-up by permitting the reaction mixture to cool or concentrating it in vacuo and collecting the precipitate by filtration. In the second step the ring-opened intermediate is ring-closed by suspending the precipitate obtained in the first step in ethanol which contains an appropriate amount of hydrobromic acid and heating the mixure slowly from 18°C to reflux temperature within 30 minutes.

Scheme 2

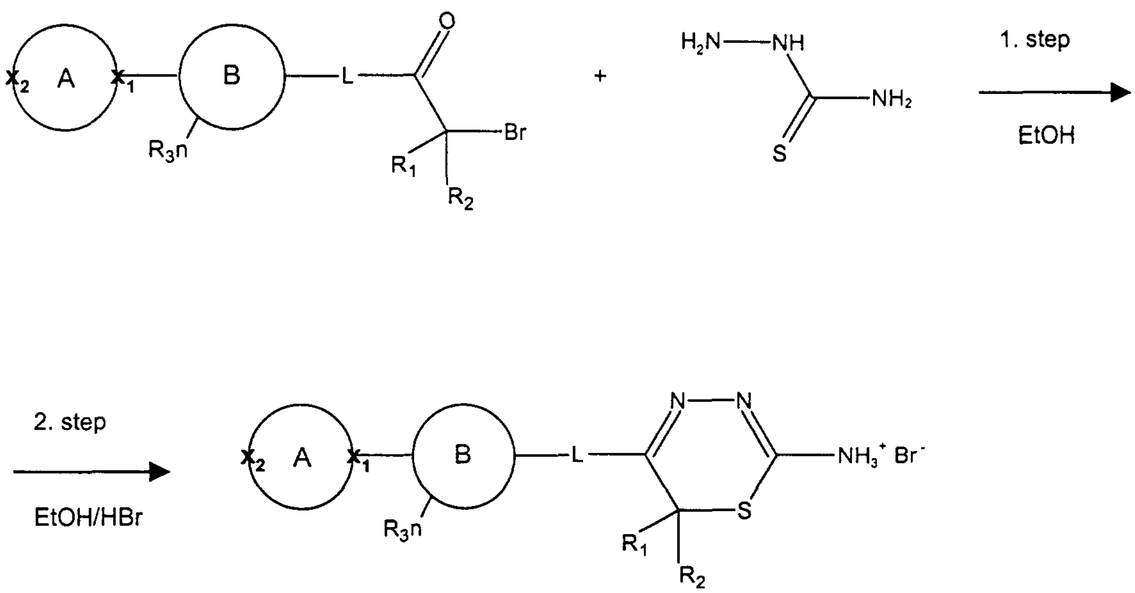


In the case of substituted phenacylchlorides the thiadiazines are obtained in good yield by heating the appropriate phenacylchloride carefully with thiosemicarbazide hydrochloride in methanol for 10 min.

Scheme 3

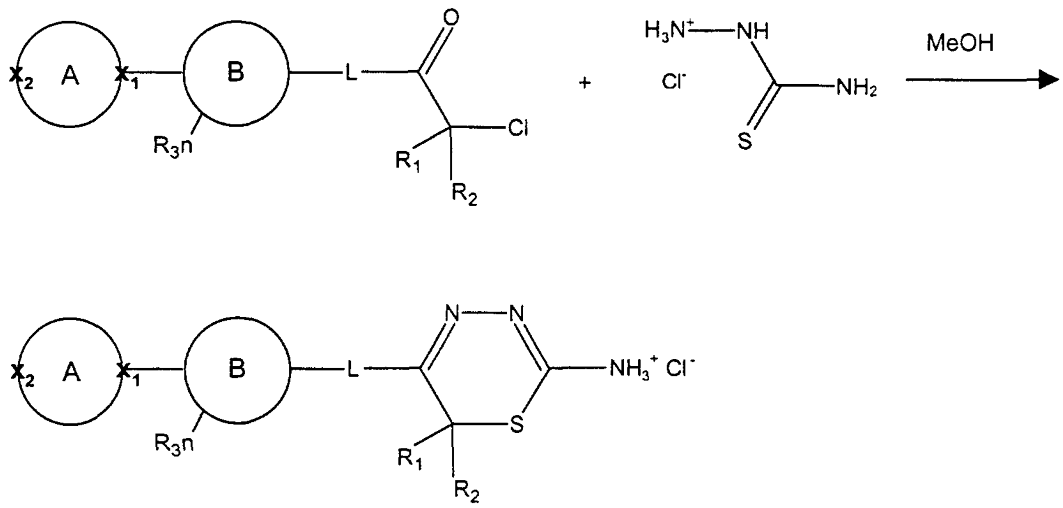


The products are generally worked-up by permitting the reaction mixture to cool or concentrating it in vacuo. The resultant residues are recrystallized from an appropriate solvent, e.g., a mixture of a lower alkanol with, e.g., acetone, acetonitrile or ethyl acetate, e.g., methanol/ acetonitrile or methanol/ethyl acetate, producing the preferred 1,3,4-thiadiazine amines or compounds of formula (**I**) as their hydrohalide salts.

The substituted phenacylhalides, the substituted carboxylic acids, the substituted sulphonylchlorides, the substituted sulphonamides of amino acids, the 4-substituted thiosemicarbazides, thiosemicarbazide and thiosemicarbazide hydrochloride which are employed as starting materials in the preparation of the compounds of formula (**I**) are either commercially available or, when unavailable, they are very readily preparable by standard chemical reactions which are well-known to those of ordinary skill in the art. For example, the substituted phenacyl halides may be prepared starting from substituted arylalkanones by the reaction with a suitable halogenating agent[29]. The substituted sulphonylchlorides may be prepared from substituted sulphonic acids according the procedure described[30]. The sulphonamides of amino acids may be prepared starting from natural or unnatural amino acids by the reaction with a substituted sulphonyl halide, preferably a substituted sulphonylchloride in the presence of potassium carbonate. The 4-substituted thiosemicarbazides may be prepared by conventionally reacting the appropriate substituted isothiocyanate with hydrazine in the presence, e.g. of diethylether[31].

EXAMPLES

Without further elaboration, it is believed that one skilled in the art can, using the preceding description, utilize the present invention to its fullest extent. The following specific embodiments are, therefore, to be construed as merely illustrative, and not limitative of the remainder of the disclosure in any way whatsoever.

General Procedures

All reactions were performed in oven-dried glassware under a positive pressure of argon and were stirred magnetically. Sensitive liquids and solutions were transferred via syringe or cannula and were introduced into reaction vessels through rubber septa.

Materials

P.a. grade reagents and solvents were used without further purification except that methylene chloride was destilled under argon from calcium hydride, and DMF was destilled under argon on molecular sieve 3 Å. Many of the specialty organic starting materials and reagents were obtained from sigma-aldrich, Deisenhofen, and lancaster-synthesis, Mühlheim. Solvents are generally obtained from merck, Darmstadt and baker, Groß-Gerau.

Chromatography

Analytical TLC was performed on Alugram Sil G/$UV_{254}$ pre-coated aluminium-backed silica gel plates (macherey-nagel, Düren). Visualization of spots was effected by one of the following techniques: (a) ultraviolet illumination and (b) immersion of the plate in a 3% solution of ninhydrin in ethanol followed by heating.

Instrumentation

Melting points (mp) were determined with a Büchi 510 melting point apparatus and are uncorrected.

Proton ($^1H$) nuclear magnetic resonance (NMR) spectra were measured with a Bruker DRX-500 (500 MHz) spectrometer, and carbon thirteen ($^{13}C$) NMR spectra were measured with a Bruker DRX-500 (125.8 MHz) spectrometer, both with TMS as an external standard. All of the compounds synthesized in the experiments below were analyzed by NMR, and the spectra were consistent with the proposed structures in each case.

Mass spectral (MS) data were obtained on a Fisons Autospec VG spectrometer by the DCI/methane and DEI/isobutane method. MALDI-TOF mass spectral data were obtained on a PerSeptive Biosystems Voyager-DE spectrometer with DHB as the matrix and PEG 200 as the calibration standard. Most of the compounds synthesized in the experiments below were analyzed by mass spectrometry, and the spectra were consistent with the proposed structures in each case.

Elemental analysis were performed on a Leco CHNS-932 elemental analyser. All inhibitors synthesized in the experiments below were analyzed by elemental analysis and most of the compounds had values within the acceptable range of +/- 0.3% for CHN.

Example 1

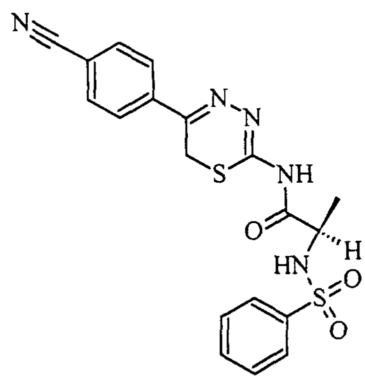

(2R)-N-[5-(4-Cyanophenyl)-6H-1,3,4-thiadiazine-2-yl]-2-[(phenylsulfonyl)amino]-propanamide

Example 1A

**General procedure for the preparation of 2-amino-6H-1,3,4-thiadiazine hydrohalides**

The following procedure was used to prepare the 2-amino-6H-1,3,4-thiadiazine hydrohalides starting from substituted phenacylbromides. The preparation of 2-amino-5-(4-cyanophenyl)-6H-1,3,4-thiadiazine hydrobromide is given as a representative example.

2-Amino-5-(4-cyanophenyl)-6H-1,3,4-thiadiazine hydrobromide

4-Cyanophenacylbromide (2.24 g, 10 mmol) and thiosemicarbazide (0.91 g, 10 mmol) were suspended in 30 mL ethanol at 0°C. The mixture was allowed to warm up to room temperature overnight with stirring. The resulting yellow slurry was cooled to -20°C and the precipitate was collected by filtration, washed with cold ethanol, and dried in vacuo. The yellow solid was again suspended in 20 mL ethanol which contained 1mL of concentrated hydrobromic acid. The mixture was heated to reflux for 30 min and was then cooled down to room temperatur overnight. The precipitate was filtered, recrystallized from ethanol, and dried in high-vacuo at 40°C over phosphorus pentoxide. Yield:

1.80 g (61%); yellow needles; mp: 248°C; $^1$H-NMR (500 MHz, DMSO-$d_6$): δ 4.35 (s, 2H), 8.00 (d, $^3J$ = 8.4 Hz, 2H), 8.05 (d, $^3J$ = 8.4 Hz, 2H), 10.19 (br s, 1H); $^{13}$C-NMR (125.8 MHz, DMSO-$d_6$): δ 22.08 ($CH_2$), 113.41, 118.30 (C), 127.74, 132.92 ($CH_{arom.}$), 137.30, 149.68, 164.22 (C). DEI MS: *m*/*z* 216 [M - HBr]$^+$.

Example 1B

**General Procedure for the preparation of phenylsulphonylated amino acids**

The following procedure was used to prepare the phenylsulphonylated amino acids described herein. The preparation of (2R)-2-[(phenylsulphonyl)amino]propanoic acid is given as a representative example.

(2R)-2-[(Phenylsulphonyl)amino]propanoic acid

Benzenesulphonylchloride (1.80 g, 10 mmol) was added to a solution of D-alanine (1.07 g, 12 mmol) in aqueous potassium carbonate (20 mL, 1.1 M). Under vigorous stirring, the mixture was carefully heated to 70°C for 30 min. The resulting clear solution was cooled in an ice bath and then acidified to pH 2.5 by the dropwise addition of concentrated hydrochloric acid with stirring. The precipitate was collected by filtration and washed with a minimum amount of ice-cold water. Recrystallization from destilled water yielded (1.65 g, 72%) (2R)-2-[(phenylsulphonyl)amino]propanoic acid as a white crystalline solid; mp: 124-126°C; $^1$H-NMR (500 MHz, DMSO-$d_6$): δ 1.13 (d, $^3J$ = 7.1 Hz, 3H), 3.76 (m, 1H), 7.54-7.62 (m, 3H), 7.78 (d, $^3J$ = 7.5 Hz, 2H), 8.15 (d, $^3J$ = 8.3 Hz, 1H), 12.65 (s, 1H); $^{13}$C-NMR (125.8 MHz, DMSO-$d_6$): δ 18.45 ($CH_3$), 51.16 (CH), 126.41, 129.07, 132.36 ($CH_{arom.}$), 141.34 (C), 173.25 (COOH).

Example 1

**General Procedure for the preparation of substituted arylthiadiazine-amides**

The following procedure was used to prepare the substituted arylthiadiazine-amides described herein. The preparation of (2R)-N-[5-(4-cyanophenyl)-6H-1,3,4-thiadiazine-2-yl]-2-[(phenyl-sulfonyl)amino]propanamide is given as a representative example.

(2R)-N-[5-(4-Cyanophenyl)-6H-1,3,4-thiadiazine-2-yl]-2-[(phenylsulfonyl)amino]-propanamide

To a suspension of 2-amino-5-(4-cyanophenyl)-6H-1,3,4-thiadiazine hydrobromide (410 mg, 1.38 mmol), (2R)-2-[(phenylsulphonyl)amino]propanoic acid (321 mg, 1.40 mmol), and 1-hydroxybenzotriazole (190 mg, 1.40 mmol) in 5 mL of DMF at 0°C was added 4-methylmorpholine (190 μL, 1.72 mmol) followed by the addition of N-(3-dimethylaminopropyl)-N'-ethyl-carbodiimide hydrochloride (275 mg, 1.43 mmol). After 48 h at 5°C, the mixture was diluted with 20 mL of 0.1 M hydrochloric acid and stirred for 30 min at room temperature. The precipitate was filtered and washed successively with water, ice-cold methanol and diethylether. After drying in vacuo the yellow solid was recystallized from methanol/acetonitrile (5 : 1) to afford 386 mg (65 %) of the title compound as yellow crystals: mp: 185-186°C; $^1$H-NMR (500 MHz, DMSO-$d_6$): δ 1.15 (d, $^3J$ = 7.1 Hz, 3H), 3.69 ($AB_q$, $^2J$ = 14.6 Hz, 2H), 4.02 (m, 1H), 7.53-7.61 (m, 3H), 7.79 (d, $^3J$ = 7.2 Hz, 2H), 7.95 (d, $^3J$ = 8.4 Hz, 2H), 8.07 (d, $^3J$ = 8.4 Hz, 2H), 8.12 (d, $^3J$ = 8.1 Hz, 1H), 12.11 (br s, 1H); $^{13}$C-NMR (125.8 MHz, DMSO-$d_6$): δ 18.83 ($CH_3$), 20.89 ($CH_2$), 53.02 (CH), 112.48, 118.51 (C), 126.46, 127.69, 129.03, 132.32, 132.74 ($CH_{arom.}$), 138.55, 141.09, 146.96 (C), one (C) and (C=O) were not detected. DCI MS: *m*/*z* 428 [M + H]$^+$; MALDI-TOF-MS: *m*/*z* calcd for [M + H]$^+$: 428.1, found: 428.2; calcd for [M + Na]$^+$: 450.1, found: 450.2; calcd for [M + K]$^+$: 466.0, found: 466.1. Anal. calcd for $C_{19}H_{17}N_5O_3S_2$: C, 53.38%; H, 4.01%; N, 16.38%. Found: C, 53.42%; H, 4.05%; N, 16.41%.

Example 5

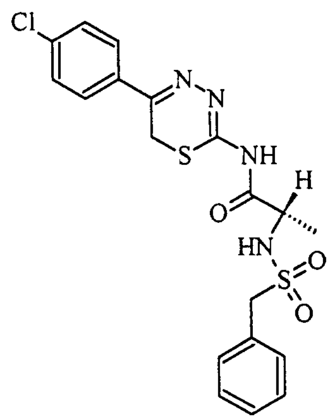

(2S)-2-[(Benzylsulfonyl)amino]-N-[5-(4-chlorophenyl)-6H-1,3,4-thiadiazin-2-yl]propanamide

Example 5A

2-Amino-5-(4-chlorophenyl)-6H-1,3,4-thiadiazine hydrobromide

The title compound was prepared starting from 4-chlorophenacylbromide as described for example **1A.** Yield: 1.84 g (60%); colourless needles; mp: 227°C; $^1$H-NMR (500 MHz, DMSO-$d_6$): δ 4.31 (s, 2H), 7.59 (d, $^3J$ = 8.6 Hz, 2H), 7.90 (d, $^3J$ = 8.7 Hz, 2H), 9.46 (br s, 1H), 10.11 (br s, 1H), 13.32 (br s, 1H); $^{13}$C-NMR (125.8 MHz, DMSO-$d_6$): δ 22.10 ($CH_2$), 128.81, 129.11 ($CH_{arom.}$), 131.86, 136.22, 150.24, 164.17 (C). DEI MS: *m*/*z* 225 [M - HBr]$^+$.

Example 5B

(2S)-2-[(Benzylsulphonyl)amino]propanoic acid

The compound was obtained by the reaction of benzylsulphonyl chloride and L-alanine according to the general procedure described for example **1B** as a white solid (1.97 g, 81%); mp: 125-127°C; $^1$H-NMR (500 MHz, DMSO-$d_6$): 1.23 (d, $^3J$ = 7.3 Hz, 3H), 3.79 (m, 1H), 4.33 ($AB_q$, $^2J$ = 13.7 Hz, 2H), 7.33-7.39 (m, 5H), 7.55 (d, $^3J$ = 7.9 Hz, 1H), 12.71 (s, 1H); $^{13}$C-NMR (125.8 MHz, DMSO-$d_6$): δ 18.67 ($CH_3$), 51.30 (CH), 58.55 ($CH_2$), 128.01, 128.26 ($CH_{arom.}$), 130.40 (C), 130.91 ($CH_{arom.}$), 174.20 (COOH).

Example 5

(2S)-2-[(Benzylsulfonyl)amino]-N-[5-(4-chlorophenyl)-6H-1,3,4-thiadiazin-2-yl]propanamide

The title compound was prepared as described for example **1**. Yield: 288 mg (46%); colourless needles; mp: 169-170°C; $^1$H-NMR (500 MHz, DMSO-$d_6$): δ 1.26 (d, $^3J$ = 7.1 Hz, 3H), 3.75 ($AB_q$, $^2J$ = 13.8 Hz, 2H), 4.00 (m, 1H), 4.33 ($AB_q$, $^2J$ = 14.0 Hz, 2H), 7.33-7.39 (m, 5H), 7.50 (d, $^3J$ = 6.2 Hz, 1H), 7.56 (d, $^3J$ = 8.5 Hz, 2H), 7.93 (d, $^3J$ = 8.4 Hz, 2H), 12.12 (br s, 1H); $^{13}$C-NMR (125.8 MHz, DMSO-$d_6$): δ 19.18 ($CH_3$), 21.19 ($CH_2$), 53.12 (CH), 58.64 ($CH_2$), 127.98, 128.24, 128.74, 128.90 ($CH_{arom.}$), 130.32 (C), 130.86 ($CH_{arom.}$), 133.10, 135.20, 147.41 (C), one (C) and (C=O) were not detected. DCI MS: *m*/*z* 451 [M + H]$^+$; MALDI-TOF-MS: *m*/*z* calcd for [M + H]$^+$: 451.1, found: 451.3; calcd for [M + Na]$^+$: 473.1, found: 473.3; calcd for [M + K]$^+$: 489.0, found: 489.3. Anal. calcd for $C_{19}H_{19}ClN_4O_3S_2$: C, 50.60%; H, 4.25%; N, 12.42%. Found: C, 50.67%; H, 3.90%; N, 12.49%.

Example 7

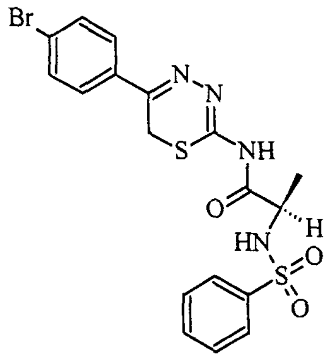

(2R)-N-[5-(4-Bromophenyl)-6H-1,3,4-thiadiazin-2-yl]-2-[(phenylsulfonyl)amino]propanamide

Example 7A

2-Amino-5-(4-bromophenyl)-6H-1,3,4-thiadiazine hydrobromide

The title compound was prepared starting from 4-bromophenacylbromide as described for example **1A**. Yield: 2.33 g (66%); colourless crystals; mp: 218°C; $^1$H-NMR (500 MHz, DMSO-$d_6$): δ 4.30 (s, 2H), 7.73 (d, $^3J$ = 8.6 Hz, 2H), 7.83 (d, $^3J$ = 8.6 Hz, 2H), 10.09 (br s, 1H); $^{13}$C-NMR (125.8 MHz, DMSO-$d_6$): δ 22.04 ($CH_2$), 125.16 (C), 128.96, 132.03 ($CH_{arom.}$), 132.21, 150.34, 164.16 (C). DEI MS: *m/z* 269 [M - HBr]$^+$.

Example 7

(2R)-N-[5-(4-Bromophenyl)-6H-1,3,4-thiadiazin-2-yl]-2-[(phenylsulfonyl)amino]propanamide

The title compound was prepared as described for example **1** using example **7A** and example **1B** as the starting reagents. Yield: 365 mg (55%); colourless crystals; mp: 179-180°C; $^1$H-NMR (500 MHz, DMSO-$d_6$): δ 1.15 (d, $^3J$ = 7.1 Hz, 3H), 3.65 ($AB_q$, $^2J$ = 14.6 Hz, 2H), 4.00 (m, 1H), 7.53-7.61 (m, 3H), 7.69 (d, $^3J$ = 8.4 Hz, 2H), 7.78 (d, $^3J$ = 7.3 Hz, 2H), 7.84 (d, $^3J$ = 8.4 Hz, 2H), 8.10 (d, $^3J$ = 8.0 Hz, 1H), 12.05 (br s, 1H); $^{13}$C-NMR (125.8 MHz, DMSO-$d_6$): δ 18.89 ($CH_3$), 21.01 ($CH_2$), 53.07 (CH), 124.05 (C), 126.46, 128.98, 129.03, 131.80, 132.31 ($CH_{arom.}$), 133.48, 141.11, 147.45 (C), one (C) and (C=O) were not detected. DCI MS: *m/z* 481 [M + H]$^+$; MALDI-TOF-MS: *m/z* calcd for [M + H]$^+$: 481.0, found: 481.0; calcd for [M + Na]$^+$: 503.0, found: 503.0; calcd for [M + K]$^+$: 519.0, found: 519.0. Anal. calcd for $C_{18}H_{17}BrN_4O_3S_2$: C, 44.91%; H, 3.56%; N, 11.64%. Found: C, 45.24%; H, 3.62%; N, 11.40%.

Example 8

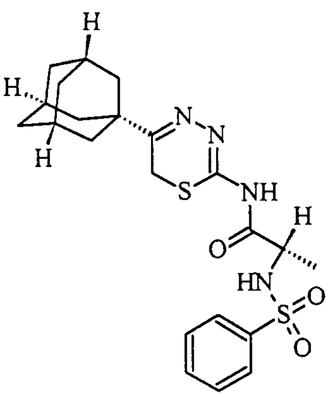

(2S)-N-[5-(1-Adamantyl)-6H-1,3,4-thiadiazin-2-yl]-2-[(phenylsulfonyl)amino]propanamide

Example 8A

Thiosemicarbazide hydrobromide

This compound was obtained as colourless crystals by evaporation of a hydrobromic acid solution of thiosemicarbazide.

Example 8B

2-Amino-5-(2-adamantyl)-6H-1,3,4-thiadiazine hydrobromide

A suspension of 1-(2-Bromoacetyl)-adamantane 2.57 g (10 mmol) and thiosemicarbazide hydrobromide 1.72 g (10 mmol) in 25 mL methanol was heated to reflux for 15 min. After cooling down to room temperature the white crystalline solid was filtered, recrystallized from methanol, and dried in high-vacuo at 40°C over phosphorus pentoxide. Yield: 1.97 g (60%); colourless crystals; mp: 251°C; $^1$H-NMR (500 MHz, DMSO-$d_6$): δ 1.61-1.68 (m, 6H), 1.75 (s, 6H), 1.98 (s, 3H), 3.79 (s, 2H), 9.11 (br s, 1H), 9.84 (br s, 1H), 12.91 (br s, 1H); $^{13}$C-NMR (128.5 MHz, DMSO-$d_6$): δ 20.39 ($CH_2$), 27.21 (CH), 35.82 ($CH_2$), 38.18 (C), 162.87 (C), 164.84 (C). DEI MS: *m*/*z* 249 [M - HBr]$^+$.

Example 8C

(2S)-2-[(Phenylsulphonyl)amino]propanoic acid

The compound was obtained by the reaction of benzenesulphonyl chloride and L-alanine according to the general procedure described for example **1B** as a white solid (1.42 g, 62%); mp: 123-125°C; $^1$H-NMR (500 MHz, DMSO-*d*$_6$): δ 1.13 (d, $^3J$ = 7.2 Hz, 3H), 3.76 (m, 1H), 7.54-7.61 (m, 3H), 7.78 (d, $^3J$ = 7.4 Hz, 2H), 8.14 (d, $^3J$ = 8.3 Hz, 1H), 12.64 (s, 1H); $^{13}$C-NMR (125.8 MHz, DMSO-*d*$_6$): δ 18.43 ($CH_3$), 51.15 (CH), 126.40, 129.06, 132.35 ($CH_{arom.}$), 141.34 (C), 173.22 (COOH).

Example 8

(2S)-N-[5-(1-Adamantyl)-6H-1,3,4-thiadiazin-2-yl]-2-[(phenylsulfonyl)amino]propanamide

The title compound was prepared as decribed for example **1**. Yield: 420 mg (66%); colourless prisms; mp: 183-184°C; $^1$H-NMR (500 MHz, DMSO-$d_6$): δ 1.11 (d, $^3J$ = 7.1 Hz, 3H), 1.64-1.70 (m, 6H), 1.77 (s, 6H), 1.99 (s, 3H), 3.17 (ABq, $^2J$ = 14.5 Hz, 2H), 3.91 (m, 1H), 7.51-7.59 (m, 3H), 7.77 (d, $^3J$ = 7.2 Hz, 2H), 7.95 (d, $^3J$ = 7.5 Hz, 1H), 11.89 (br s, 1H); $^{13}$C-NMR (125.8 MHz, DMSO-$d_6$): δ 18.99 ($CH_3$), 20.07 ($CH_2$), 27.32 (CH), 35.95 ($CH_2$), 38.55 (C), 53.59 (CH), 126.38, 128.91, 132.14 ($CH_{arom.}$), 141.19, 159.80 (C), one (C) and (C=O) were not detected. DCI MS: *m*/

z 461 [M + H]$^+$; MALDI-TOF-MS: m/z calcd for [M + H]$^+$: 461.2, found: 461.0; calcd for [M + Na]$^+$: 483.2, found: 483.1. Anal. calcd for $C_{22}H_{28}N_4O_3S_2$: C, 57.37%; H, 6.13%; N, 12.16%. Found: C, 57.19%; H, 6.24%; N, 11.92%.

Example 30

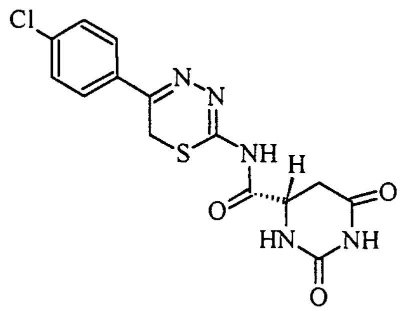

(4S)-N-[5-(4-Chlorophenyl)-6H-1,3,4-thiadiazin-2-yl]-2,6-dioxohexahydro-4-pyrimidinecarboxamide

The title compound was prepared as described for example **1** using example **5A** and L-hydroorotic acid as the starting reagents. Yield: 238 mg (47%) from methanol; colourless crystals; mp: 215°C (dec.); $^1$H-NMR (500 MHz, DMSO-$d_6$): δ 2.59-2.63 (m, 1H), 2.91-2.96 (m, 1H), 3.79 ($AB_q$, $^2J$ = 14.9 Hz, 2H), 4.18 (m, 1H), 7.55 (d, $^3J$ = 8.6 Hz, 2H), 7.64 (s, 1H), 7.91 (d, $^3J$ = 8.6 Hz, 2H), 10.08 (s, 1H), 12.46 (br s, 1H); $^{13}$C-NMR (125.8 MHz, DMSO-$d_6$): δ 21.27, 33.21 ($CH_2$), 51.54 (CH), 128.72, 128.92 ($CH_{arom.}$), 132.86, 135.32, 147.52, 153.61, 169.27 (C), one (C) and (C=O) were not detected. DCI MS: m/z 366 [M + H]$^+$; MALDI-TOF-MS: m/z calcd for [M + H]$^+$: 366.0, found: 366.2; calcd for [M + Na]$^+$: 388.0, found: 388.2. Anal. calcd for $C_{14}H_{12}ClN_5O_3S$: C, 45.97%; H, 3.31%; N, 19.15%. Found: C, 46.20%; H, 2.98%; N, 19.17%.

PHARMACEUTICAL COMPOSITION EXAMPLES

The compounds of the invention are useful to prepare compositions for the treatment of ailments and the like. The following composition examples do not limit the invention, but provide guidance to the skilled artisan to prepare and use the compounds and compositions of the invention.

Example A

A tablet composition for oral administration, according to the present invention, is made comprising:

| Component | Amount (%w/w) |
|---|---|
| Example 21 | 9 |
| Lactose | 56 |
| Maize Starch | 33 |
| Talcum | 1 |
| Magnesium Stearate | 1 |

The above ingredients with the exception of the magnesium stearate are combined and granulated using water as a granulating liquid. The formulation is then dried, mixed with the magnesium stearate and formed into tablets with an appropriate tablet machine.

Example B

A suppository form for rectal administration, according to the present invention, is made comprising:

| Component | Amount (%w/w) |
|---|---|
| Example 27 | 11 |
| Polyethylene glycol 1000 | 69.5 |
| Polyethylene glycol 4000 | 19.5 |

The ingredients are melted together and mixed on a steam bath, and poured into molds containing 2.5 g total weight.

Example C

A composition for parental administration is made comprising:

| Component | Amount |
|---|---|
| Example 7 | 0.02 g |
| Propylene glycol | 20.0 g |
| Polyethylene glycol 400 | 20.0 g |
| Polysorbate 80 | 1.0 g |
| 0.9% Saline solution | q.s. 100 ml |

The compound is dissolved in propylene glycol, polyethylene glycol 400 and polysorbate 80. A sufficient quantity of 0.9% saline solution is then added with stirring to provide 100 ml of the i.V. solution which is filtered through a 0.2 μ membrane filter and packaged under sterile conditions.

Example D

A inhalation aerosol composition, according to the present invention, is made comprising:

| Component | Composition (%w/v) |
|---|---|
| Example 11 | 5.0 |
| Ethanol | 33.0 |
| Ascorbic acid | 0.1 |
| Menthol | 0.1 |
| Sodium Saccharine | 0.2 |
| Propellant (F12, F114) | q.s. 100% |

The compound is dispersed in ethanol and the propellants and mixed with the other ingredients. The resulting mixture is then poured into an aerosol container fitted with a metering valve.

Example E

A topical sunscreen preparation, according to the present invention, is made comprising:

| Component | Amount (%w/w) |
|---|---|
| Example 20 | 3.0 |
| all-*trans* retinoic acid | 2.0 |
| Ethanol | 84.2 |
| Citric acid | 2.0 |

(continued)

| Component | Amount (%w/w) |
|---|---|
| Myristyl lactat | 5.8 |
| Silicone oil | 2.0 |
| Hydroxypropyl cellulose acetate | 1.0 |

The compound is dispersed in ethanol and mixed with the other ingredients to give a sunscreen gel.

REFERENCES

[1] Hooper, N.M.: Families of zinc metalloproteases. *FEBS Lett.* **354** (1994) 1-6.

[2] Bode, W., Fernandez-Catalan, C., **Tschesche, H.**, Grams, F., Nagase, H., and Maskos, K.: Structural properties of matrix metalloproteinases. *Cell. Mol. Life Sci.* **55** (1999) 639-652.

[3] Birkedal-Hansen, H., Moore, W.G.I., Bodden, M.K., Windsor, L.J., Birkedal-Hansen, B., DeCarlo, A., and Engler, J.A.: Matrix Metalloproteinases - A Review. *Critical Reviews In Oral Biology & Medicine* **4** (1993) 197-250.

[4] Curran, S. and Murray, G.I.: Matrix metalloproteinases in tumour invasion and metastasis. *J. Pathol.* **189** (1999) 300-308.

[5] Nelson, A.R., Fingleton, B., Rothenberg, M.L., and Matrisian, L.M.: Matrix Metalloproteinases: Biologic Activity and Clinical Implications. *J. Clin. Oncol.* **18** (2000) 1135-1149.

[6] Vu, T.H. and Werb, Z.: Matrix Metalloproteinases. 1998. Edited by Parks, W.C. and Mecham, R.P., *Academic Press* (1998).

[7] Friers, W.: Tumor necrosis factor: Characterization at the molecular, cellular and *in vivo* level. *FEBS Lett.* **285** (1991) 199-212.

[8] Kuno, K., Kanada, N., Nakashima, E., Fujiki, F., Ichimura, F. and Matsushima, K.: Molecular Cloning of a Gene Encoding a New Type of Metalloproteinase-disintegrin Family Protein with Thrombospondin Motifs as an Inflammation Associated Gene. *J. Biol. Chem.* **272** (1997) 556-562.

[9] Wu, E., Croucher, P.I. and McKie, N.: Expression of members of the novel membrane linked metalloproteinase family ADAM in cells derived from a range of haematological malignancies. *Biochem Biophys. Res. Comm.* **235** (1997) 437-442.

[10] Beeley, N.R.A., Ansell, P.R.J., and Docherty, A.J.P.: Inhibitors of matrix metalloproteinases (MMPs). *Curr. Opin. Ther. Patents* **4** (1994) 7-16.

[11] Becket, R.P., Davidson, A.H., Drummond, A.H., Huxley, P., and Whittaker, M.: Recent advantages in matrix metalloproteinase inhibitor research. *Drug Discov. Today* **1** (1996) 16-27.

[12] Baici, A.: Inhibition of extracellular matrix-degrading endopeptidases: Problems, comments, and hypothesis. *Biol. Chem.* **379** (1998) 1007-1018.

[13] Denis, L.J. and Verweij, J.: Matrix metalloproteinase inhibitors: Present achievements and future prospects. *Invest. New Drugs* **15** (1997) 175-185.

[14] Becket, R.P. and Whittaker, M.: Matrix metalloproteinase inhibitors 1998. *Exp. Opin. Ther. Patents* **8** (1998) 259-282.

[15] Brown, P.D.: Matrix metalloproteinase inhibitors: A new class of anticancer agent. *Curr. Opin. Invest. Drugs* **2** (1993) 617-626.

[16] Publication *J. Med. Chem.,* submitted.

[17] Berge, S.M., Bighley, L.D., and Monkhouse, D.C.: Pharmaceutical salts. *J. Pharmaceutical Sciences* **66** (1977) 1-19.

[18] Fisher, G.J., Talwar, H.S., Lin, J., and Voorhees, J.J.: Molecular mechanisms of photoaging in human skin *in vivo* and their prevention by all-*trans* retinoic acid. *Photochemistry and Photobiology* **69** (1999) 154-157.

[19] Kröger, M. and **Tschesche, H.**: Cloning, expression and activation of a truncated 92-kDa gelatinase minienzyme. *Gene* **196** (1997) 175-180.

[20] Kleine, T., Bartsch, S., Bläser, J., Schnierer, S., Triebel, S., Valentin, M., Gote, T., and **Tschesche, H.**: Preparation of active recombinant TIMP-1 from *Escherichia-Coli* inclusion-bodies and complex-formation with the recombinant catalytic domain of PMNL-collagenase. *Biochemistry* **32** (1993) 14125-14131.

[21] **Tschesche, H.**, Knäuper, V., Krämer, S., Michaelis, J., Oberhoff, R., and Reinke, H.: Latent collagenase and gelatinase from human neutrophils and their activation. *Matrix* **1** (1992) 245-255.

[22] Pieper, M., Betz, M., Budisa, N., Gomis-Rüth, F.X., Bode, W., and **Tschesche, H.**: Expression, purification, characterization, and x-ray analysis of selenomethionine 215 variant of leukocyte collagenase. J. *Protein Chem.* **16** (1997) 637-650.

[23] Hiller, O., Lichte, A., Oberpichler, A., Kocourek, A., and **Tschesche, H.**: Matrix metalloproteinases Collagenase-2, Macrophage Elastase, Collagenase-3, and Membrane Type-1 matrix metalloproteinase impair clotting by degradation of fibrinogen and factor XII. *J. Biol. Chem.,* published August 4, 2000 as manusscript M001836200.

[24] Lichte, A., Kolkenbrock, H., and **Tschesche, H.**: The recombinant catalytic domain of membrane-type matrix metalloproteinase-1 (MT1-MMP) induces activation of progelatinase A and progelatinase A complexed with TIMP-2. *FEBS Lett.* **397** (1996) 277-282.

[25] Roderfeld, M., Buttner, F.H., Bartnik, E., and **Tschesche, H.**: Expression of human membrane type 1 matrix metalloproteinase in *Pichia pastoris*. *Protein Expr. Purif.* **19** (2000) 369-374.

[26] Knight, C.G., Willenbrock, F., and Murphy, G.: A novel coumarin-labelled peptide for sensitive continous assays of the matrix metalloproteinases. *FEBS Lett.* **296** (1992) 263-266.

[27] Cheng, Y.C. and Prusoff, W.H.: Relationship between the inhibition constant ($K_i$) and the concentration of inhibitor which causes 50 per cent inhibition ($I_{50}$) of an enzymatic reaction. *Biochem. Pharm.* **22** (1973) 3099-3108.

[28] Schwettmann, L. and **Tschesche, H.**: Cloning and Expression of Catalytically Active Metalloproteinase Domain of ADAM9 in *Pichia pastoris*. *Protein Expr. Purif.* **0114** (2000).

[29] *Houben-Weyl,* Vol. **E4** (1960) 171-189.

[30] Corey, E.J. and Posner, G.H.: Formation of olefins via pyrolysis of sulfonate esters. *J. Org. Chem.* **54** (1989) 389-393.

[31] Jensen, K.A., Anthoni, U., Kägi, B., Larsen, C., and Pedersen, C.T.: Studies of thioacids and their derivatives. *Acta Chem. Scan.* **22** (1968) 1-50.

## Claims

**1.** A compound having a structure according to formula (I)

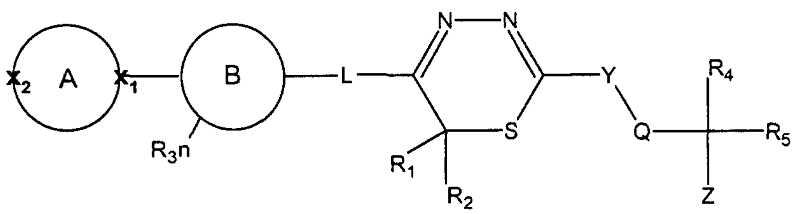

(I)

wherein:

| | |
|---|---|
| Ring A | is a 5-7 membered aliphatic ring and may optionally be mono- or di-substituted by optionally substituted C1-6 alkyl or C1-6 alkoxy, each substituent being independently selected from halogen, C1-6 alkyl or an oxo group; $x_1$ and $x_2$ are independently selected from N, O and C, where a ring substituent on ring A is an oxo group this is preferably adjacent a ring nitrogen atom; |
| Ring B | is a monocyclic or bicyclic alkyl, aryl, aralkyl, heteroaryl or heteroaralkyl ring comprising up to 12 ring atoms and containing no, one or up to three heteroatoms independently chosen from N, O, and S; alternatively ring A may be omitted; |
| each $R_3$ | is independently selected from hydrogen, halogen, $-NO_2$, -CN, $-CF_3$, -OH, $-NH_2$, C1-6 alkyl, -S-C1-6 alkyl, -SO-C1-6 alkyl, $-SO_2$-C1-6 alkyl, $-SO_2-NH_2$, C1-6 alkoxy, up to C10 aryloxy, and COOR wherein R is hydrogen or C1-6 alkyl, n is 1-5; |
| L | is -CO- or a direct bond; |
| $R_1$ and $R_2$ | are independently selected from hydrogen, halogen, or C1-6 alkyl; |
| Y | is nitrogen or sulphur; |

Q is selected from -CO-, $-SO_2-$, $-CR_6R_7-$, wherein $R_6$ and $R_7$ independently selected from H, -OH-, C1-6 alkyl, C5-7 cycloalkyl, up to C10 aryl, up to C10 heteroaryl, up to C12 aralkyl, or up to C12 heteroarylalkyl;

$R_4$ is H, C1-6 alkyl, C5-7 cycloalkyl, up to C10 aryl, up to C10 heteroaryl, up to C12 aralkyl, or up to C12 heteroarylalkyl, all optionally substituted by up to three groups independently selected from $-NO_2$, -OH, $-CF_3$, $-NH_2$, $-CO-NH_2$, -COOH, halogen, C1-4 alkyl, C1-4 alkyloxy, carboxy (C1-4) alkyl, or up to C6 cycloalkyl;

$R_5$ is H, C1-6 alkyl, or together with $R_4$ forms a carbocyclic or heterocyclic 5, 6 or 7 membered ring, the latter containing at least one or up to three heteroatoms selected from N, O, and S and may optionally substituted by one or up to four oxo groups;

Z is a 5, 6 or 7 membered alkyl, aryl, heteroalkyl, or heteroaryl ring comprising one or up to three of O, S and N and may optionally substituted by one or up to four oxo groups; alternatively Z is a nitrogen optionally substituted by hydrogen, C1-4 alkyl, C5-7 cycloalkyl, up to C10 aryl, up to C10 heteroaryl, up to C12 aralkyl, or up to C12 heteroarylalkyl, -CO-C1-6 alkyl, -CO-C1-6 aryl, -CO-C1-6 heteroaryl, $-SO_2$-C1-6 alkyl, $-SO_2$-C1-6 aryl, $-SO_2$-C1-6 heteroalkyl, $-SO_2$-C1-6 heteroaryl, $-SO_2$-CH2-C1-6 aryl, $-SO_2$-CH2-C1-6 heteroalkyl, $-SO_2$-CH2-C1-6 heteroaryl, heteroatoms selected from N, O or S, all rings optionally substituted by up to three groups independently selected from hydrogen, halogen, -OH, -COOH, $-CH_2$-COOH, -CN, $-CF_3$, C1-6 alkyl, or C1-6 alkoxy; alternatively Z is a hydrogen.

This structure also includes an optical isomer, diastereomer or enantiomer for formula (**I**), or a pharmaceutically-acceptable salt, or biohydrolyzable amide, ester, or imide thereof.

**2.** A process for preparing a compound of the formula (**I**) comprising the acylation of a substituted 1,3,4-thiadiazine amine hydrohalide with an appropriately substituted sulphonamide of an amino acid derivative or an substituted carboxylic acid using as a coupling agent N-(3-dimethyl-aminopropyl)-N'-ethyl-carbodiimide hydrochloride (EDCI).

**3.** The compound of claim 1 which is

(2R)-N-[5-(4-Cyanophenyl)-6H-1,3,4-thiadiazin-2-yl]-2-[(phenylsulfonyl)amino]-propanamide
(2R)-N-[5-(4-Chlorophenyl)-6H-1,3,4-thiadiazin-2-yl]-3-methyl-2-[(phenylsulfonyl)amino]butanamide
(2S)-N-[5-(4-Cyanophenyl)-6H-1,3,4-thiadiazin-2-yl]-2-[(phenylsulfonyl)amino]-propanamide
(2S)-2-[(Phenylsulfonyl)amino]-N-{5-[4-(trifluormethyl)phenyl]-6H-1,3,4-thiadiazin-2-yl}-propanamide
(2S)-2-[(Benzylsulfonyl)amino]-N-[5-(4-chlorophenyl)-6H-1,3,4-thiadiazin-2-yl]-propanamide
(2S)-N-[5-(4-Bromophenyl)-6H-1,3,4-thiadiazin-2-yl]-2-[(phenylsulfonyl)amino]-propanamide
(2R)-N-[5-(4-Bromophenyl)-6H-1,3,4-thiadiazin-2-yl]-2-[(phenylsulfonyl)amino]-propanamide
(2S)-N-[5-(1-Adamantyl)-6H-1,3,4-thiadiazin-2-yl]-2-[(phenylsulfonyl)amino]-propanamide
(2S)-N-[5-(4-Nitrophenyl)-6H-1,3,4-thiadiazin-2-yl]-2-[(phenylsulfonyl)amino]-propanamide
(2S)-N-[5- (5-Chloro-2-thienyl)-6H-1,3,4-thiadiazin-2-yl]-2-[(phenylsulfonyl)-amino]propanamide
(2S)-N-[5-(4-Chlorophenyl)-6H-1,3,4-thiadiazin-2-yl]-2-[(phenylsulfonyl)amino]-propanamide
(2S)-N-[5-(4-Methoxyphenyl)-6H-1,3,4-thiadiazin-2-yl]-2-[(phenylsulfonyl)-amino]propanamide
(2S)-N-[5-(4-Fluorophenyl)-6H-1,3,4-thiadiazin-2-yl]-2-[(phenylsulfonyl)amino]-propanamide
(2R)-N-[5-(4-Fluorophenyl)-6H-1,3,4-thiadiazin-2-yl]-2-[(phenylsulfonyl)amino]-propanamide
(2R)-N-[5-(4-Methylphenyl)-6H-1,3,4-thiadiazin-2-yl]-2-[(phenylsulfonyl)-amino]propanamide
2-{[(4-Methoxyphenyl)sulfonyl]amino}-N-[5-(2,3,4,5,6-pentafluorophenyl)-6H-1,3,4-thiadiazin-2-yl]acetamide
(2S)-2-{[(4-Methoxyphenyl)sulfonyl]amino}-N-[5-(4-methylphenyl)-6H-1,3,4-thiadiazin-2-yl]propanamide
2-{[(4-tert-Butylphenyl)sulfonyl]amino}-N-[5-(2,4-dichlorophenyl)-6H-1,3,4-thiadiazin-2-yl]acetamide
N-[5-(2,4-Dichlorophenyl)-6H-1,3,4-thiadiazin-2-yl]-2-{[(4-methoxyphenyl)-sulfonyl]amino}acetamide
N-[5-(4-Chlorophenyl)-6H-1,3,4-thiadiazin-2-yl]-2-methyl-2-[(phenylsulfonyl)-amino]propanamide
(2R)-N-[5-(4-Chlorophenyl)-6H-1,3,4-thiadiazin-2-yl]-2-[(phenylsulfonyl)amino]-propanamide
(2S)-N-[5-(4-Chlorophenyl)-6H-1,3,4-thiadiazin-2-yl]-3-methyl-2-[(phenylsulfonyl)amino]butanamide
N-[5-(4-Chlorophenyl)-6H-1,3,4-thiadiazin-2-yl]-2-(2,5-dioxo-4-imidazolidinyl)acetamide
N-[5-(4-Bromophenyl)-6H-1,3,4-thiadiazin-2-yl]-2-(2,5-dioxo-4-imidazolidinyl)acetamide
N-[5-(4-Cyclohexylphenyl)-6H-1,3,4-thiadiazin-2-yl]-2-(2,5-dioxo-4-imidazolidinyl)-acetamide
2-(2,5-Dioxo-4-imidazolidinyl)-N-{5-[4-(1-pyrrolidinyl)phenyl]-6H-1,3,4-thiadiazin-2-yl}-acetamide
(2S)-N-[5-(4-Chlorophenyl)-6H-1,3,4-thiadiazin-2yl-]-2-[(2-thienylsulfonyl)amino]-propanamide

(2S)-N-[5-(4-Chlorophenyl)-6H-1,3,4-thiadiazin-2yl-]-2-[(4-morpholinylsulfonyl)amino]-propanamide
(2S)-N-[5-(4-Chlorophenyl)-6H-1,3,4-thiadiazin-2-yl]-2-[(3-pyridylsulfonyl)amino]-propanamide
(4S)-N-[5-(4-Chlorophenyl)-6H-1,3,4-thiadiazin-2-yl]-2,6-dioxohexahydro-4-pyrimidine-carboxamide
N-Allyl-5-(4-chlorophenyl)-6H-1,3,4-thiadiazin-2-amine hydrobromide and pharmaceutically acceptable salts thereof.

**4.** The use of a compound of any preceding claim as an inhibitor of matrix metalloproteinases.

**5.** The use of a compound of any preceding claim as an inhibitor of a mammalian reprolysin.

**6.** The use of a compound of any preceding claim as a therapeutically active substance.

**7.** A pharmaceutical composition for the treatment of a condition selected from the group consisting of arthritis inflammatory bowel disease, Crohn's disease, cancer, tissue ulceration, restenosis, periodontal disease, epidermolysis bullosa, osteoporosis, loosening of artificial joint implants, atherosclerosis, aortic aneurysm, congestive heart failure, myocardial infarction, stroke, cerebral ischemia, head trauma, spinal cord injury, neuro-degenerative disorders, autoimmune disorders, Huntington's disease, Parkinson's disease, migraine, depression, emphysema, chronic obstructive pulmonary disease, Alzheimer's disease, organ transplant toxicity, cachexia, allergic reactions, allergic contact hypersensitivity, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetes, tumor invasion, tumor growth, tumor metastasis, corneal scarring, psoriasis, scleritis, AIDS, sepsis and septic shock in a mammal, comprising an amount of a compound of any preceding claim effective in such treatment and a pharmaceutically acceptable carrier.

**8.** A method for treating a condition selected from the group consisting of arthritis inflammatory bowel disease, Crohn's disease, cancer, tissue ulceration, restenosis, periodontal disease, epidermolysis bullosa, osteoporosis, loosening of artificial joint implants, atherosclerosis, aortic aneurysm, congestive heart failure, myocardial infarction, stroke, cerebral ischemia, head trauma, spinal cord injury, neuro-degenerative disorders, autoimmune disorders, Huntington's disease, Parkinson's disease, migraine, depression, emphysema, chronic obstructive pulmonary disease, Alzheimer's disease, organ transplant toxicity, cachexia, allergic reactions, allergic contact hypersensitivity, peripheral neuropathy, pain, cerebral amyloid angiopathy, nootropic or cognition enhancement, amyotrophic lateral sclerosis, multiple sclerosis, ocular angiogenesis, corneal injury, macular degeneration, abnormal wound healing, burns, diabetes, tumor invasion, tumor growth, tumor metastasis, corneal scarring, psoriasis, scleritis, AIDS, sepsis and septic shock in a mammal, comprising administering to said mammal an amount of a compound of any preceding claim, effective in treating such a condition.

**9.** A pharmaceutical composition for the treatment of a condition which can be treated by the inhibition of matrix metalloproteinases in a mammal, comprising an amount of a compound of any preceding claim effective in such treatment and a pharmaceutically acceptable carrier.

**10.** A pharmaceutical composition for the treatment of a condition which can be treated by the inhibition of a mammalian reprolysin in a mammal, comprising an amount of a compound of any preceding claim effective in such treatment and a pharmaceutically acceptable carrier.

**11.** A method for the inhibition of matrix metalloproteinases in a mammal, comprising administering to said mammal an effective amount of a compound of any preceding claim.

**12.** A method for the inhibition of a mammalian reprolysin in a mammal, comprising administering to said mammal an effective amount of a compound of any preceding claim.

**13.** The use of a compound of any preceding claim in the treatment of a disease-state which is alleviated by treatment with a matrix metalloproteinase inhibitor, especially wherein the disease-state results from ultraviolet-B (UVB) irradiation such as skin reddening, sun-burns, skin-cancer, leathery textures, wrinkles, or mottled pigmentations.

**14.** The use of a compound of any preceding claim to prepare a sunscreen composition in conjunction with all-*trans* retinoic acid.

European Patent Office

# EUROPEAN SEARCH REPORT

Application Number
EP 00 12 0727

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 276 804 A (HOECHST AG)<br>3 August 1988 (1988-08-03)<br>* page 3, line 1 - line 3 *<br>* page 4, formula I *<br>* page 4, line 50 - line 52 *<br>* page 6, line 31 - line 41 *<br>* table 1, compounds 1, 3-15, 22, 23, 27-31 *<br>--- | 1,2,7,9, 10 | C07D285/16<br>C07D417/12<br>A61K31/54<br>A61P37/00 |
| A | EP 0 952 148 A (PFIZER PROD INC)<br>27 October 1999 (1999-10-27)<br>* page 2, line 5 - line 9 *<br>* page 2, line 49 - line 55 *<br>* page 3, formula I *<br>* page 4, line 8 - line 14 *<br>* page 5, line 1 - line 48 *<br>--- | 1-13 | |
| A | WO 98 55075 A (UNIV MICHIGAN)<br>10 December 1998 (1998-12-10)<br>* claims 1,3,8,23 *<br>----- | 1,14 | |

TECHNICAL FIELDS SEARCHED (Int.Cl.7)

A61K
C07D

The present search report has been drawn up for all claims

| Place of search | Date of completion of the search | Examiner |
|---|---|---|
| BERLIN | 5 March 2001 | Hoepfner, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 12 0727

This annex lists the patent family members relating to the patent documents cited in the above-mentioned European search report.
The members are as contained in the European Patent Office EDP file on
The European Patent Office is in no way liable for these particulars which are merely given for the purpose of information.

05-03-2001

| Patent document cited in search report | Publication date | Patent family member(s) | Publication date |
|---|---|---|---|
| EP 0276804 A | 03-08-1988 | DE 3702756 A | 11-08-1988 |
| | | AT 73785 T | 15-04-1992 |
| | | AU 602053 B | 27-09-1990 |
| | | AU 1099788 A | 04-08-1988 |
| | | CN 88100519 A,B | 28-12-1988 |
| | | DE 3869144 A | 23-04-1992 |
| | | DK 47688 A | 31-07-1988 |
| | | ES 2038216 T | 16-07-1993 |
| | | FI 880386 A | 31-07-1988 |
| | | GR 3004685 T | 28-04-1993 |
| | | HU 47263 A,B | 28-02-1989 |
| | | JP 63192765 A | 10-08-1988 |
| | | NO 880406 A,B, | 01-08-1988 |
| | | NZ 223335 A | 27-09-1989 |
| | | PH 24609 A | 17-08-1990 |
| | | PT 86656 A,B | 01-02-1988 |
| | | US 4940790 A | 10-07-1990 |
| | | ZA 8800641 A | 28-09-1988 |
| EP 0952148 A | 27-10-1999 | BR 9901250 A | 16-05-2000 |
| | | JP 11322705 A | 24-11-1999 |
| | | US 6156798 A | 05-12-2000 |
| WO 9855075 A | 10-12-1998 | AU 8057398 A | 21-12-1998 |
| | | BR 9809969 A | 17-10-2000 |
| | | CN 1259040 T | 05-07-2000 |
| | | EP 0998251 A | 10-05-2000 |
| | | NO 995768 A | 17-01-2000 |
| | | PL 337531 A | 28-08-2000 |
| | | US 6130254 A | 10-10-2000 |
| | | ZA 9804791 A | 06-01-1999 |

EPO FORM P0459

For more details about this annex : see Official Journal of the European Patent Office, No. 12/82